# EUROPÄISCHE PATENTSCHRIFT

(11) **EP 2 726 185 B1**
(45) Veröffentlichungstag und Bekanntmachung des Hinweises auf die Patenterteilung: **03.10.2018**
(21) Anmeldenummer: 12709327.6
(22) Anmeldetag: 19.03.2012
(51) Int. Cl.: B01F 17/00, C11D 17/00, C11D 3/37, A61K 8/898, A61K 8/06

(54) **MIKROEMULSION VON QUATERNÄREN AMMONIUMGRUPPEN ENTHALTENDEN POLYSILOXANEN, DEREN HERSTELLUNG UND VERWENDUNG**
MICROEMULSION OF QUATERNARY POLYSILOXANES CONTAINING AMMONIUM GROUPS, PRODUCTION AND USE THEREOF
MICROÉMULSION DE POLYSILOXANES CONTENANT DES GROUPES AMMONIUM QUATERNAIRE, PRODUCTION ET UTILISATION DE LADITE MICROÉMULSION

(30) Priorität: 30.06.2011 DE 102011078382
(43) Veröffentlichungstag der Anmeldung: 07.05.2014
(73) Patentinhaber: Evonik Degussa GmbH, 45128 Essen (DE)
(72) Erfinder: DAHL, Verena, 50858 Köln (DE); HERRWERTH, Sascha, 45134 Essen (DE); HARTUNG, Christian, 45133 Essen (DE); VENZMER, Joachim, 45239 Essen (DE); KUPPERT, Dirk, 63739 Aschaffenburg (DE); DE GANS, Berend-Jan, 45478 Mülheim an der Ruhr (DE)
(86) Internationale Anmeldenummer: PCT/EP2012/054785
(87) Internationale Veröffentlichungsnummer: WO 2013/000592

(56) Entgegenhaltungen:
- EP-A1- 1 426 398
- EP-A1- 1 887 024
- WO-A2-2011/042409
- US-A1- 2007 212 326

## Beschreibung

### Gebiet der Erfindung

Gegenstand der Erfindung sind Mikroemulsionen, welche als Öl-Phase ein mindestens eine quaternäre Ammoniumgruppe enthaltendes Polysiloxan aufweisen, Verfahren zu deren Herstellung sowie die Verwendung solcher Mikroemulsionen.

### Stand der Technik

Polysiloxane mit quaternären Gruppen und deren Anwendung als Additive für Haarpflege oder Textilweichmacher sind aus der Patentliteratur bekannt.

In der EP 1887024 mit Anmeldedatum 04.07.2007 werden endständig kationische Polysiloxane mit einer sogenannten T-Struktur und deren Verwendung als Konditioniermittel in kosmetischen Formulierungen beschrieben. Diese kationischen Polysiloxane zeigen einen ausgeprägten konditionierenden und glanzerzeugenden Effekt. Allerdings weisen die potentiell besser wirkenden höhermolekularen kationischen Polysiloxane mit dieser T-Struktur das Problem auf, dass sie extrem hochviskos sind und so nicht handhabbar und in kosmetische Formulierungen einarbeitbar sind.

Dieselbe Aussage lässt sich für die in WO2011/088937 A1 mit Anmeldedatum 17.12.2010 offenbarten, multimere T-Strukturen aufweisenden Polysiloxane treffen. Eine Möglichkeit, Polysiloxane in kosmetische Formulierungen einzuarbeiten, stellt die Darreichungsform der Polyorganosiloxane in Form von Emulsionen oder Mikroemulsionen dar. Mikroemulsionen sind thermodynamisch stabile Mischungen aus Wasser (wässrige Phase), Öl (nicht mit Wasser mischbare Phase) und Tensid (Solubilisator). Mikroemulsionen, bei denen die Öl-Phasen maßgeblich durch Polysiloxane gebildet werden, sind bekannt.

Die US 4620878 beschreibt die generelle Herstellung von Emulsionen und Mikroemulsionen, die lineare aminofunktionelle Polyorganosiloxane enthalten. Als erstes wird ein Konzentrat bestehend aus Tensid, Polyorganosiloxan und kleinen Mengen Wasser hergestellt, das dann schnell im restlichen, zur Bildung der Mikroemulsion nötigen Wasser dispergiert wird.

Die EP 0459500 beschreibt, wie polysiloxanhaltige Mikroemulsionen durch Emulsionspolymerisation hergestellt werden.

Die US 6607717 beschreibt die Herstellung und Verwendung von Emulsionen und anderen Formulierungen mit kammartigen, quaternären Polyorganosiloxanen unter Einsatz von nichtionischen ethoxylierten Emulgatoren.

Die US 4749732 beschreibt die Verwendung von aminoalkyl-substituierten Polyorganosiloxanen und ihre Darreichung als Emulsion oder Mikroemulsion für klare Hair Care Anwendungen.

US 6153569 beschreibt die Verwendung von Mikroemulsionen mit aminofunktionellen Polyorganosiloxanen, um klare Shampoo-Formulierungen zu erhalten.

Weichspülerzusammensetzungen, die im Spülgang nach der Reinigung oder Wäsche von Geweben und/oder Textilien, eingesetzt werden, sind bekannt. Weiterhin ist bekannt, dass Weichspülerzusammensetzungen eine oder mehrere Silicone oder organisch modifizierte Siloxane enthalten können, die Verknittern von Gewebe nach dem Spülgang und dem Trocknen vermindern, das Bügeln erleichtern, eine erhöhte Weichheit oder ein verbessertes Rücknetzvermögen bewirken. Offenbart wird dies zum Beispiel in den WO 9524460, FR7621830, GB 1596792, US 4426299, US 4806255, GB 0239910 und US 4855072.

Die Verwendung von Mikroemulsionen zum Einbringen von Siliconen in Weichspülerformulierungen wird zum Beispiel in der WO 92/01776 beschrieben.

Die Verwendung von Makroemulsionen zum Einbringen von Siliconen in Weichspülerformulierungen wird z. B. in den WO A 97/31997 und WO A 97/31998 beschrieben.

Wässrige Lacke, die Polyurethandispersionen als Bindemittel enthalten sind ebenfalls bekannt. Solche Dispersionen werden als Einkomponenten- oder 1K-Systeme bezeichnet und zur Beschichtung von Substraten jeglicher Art, vorzugsweise Textil, Metall, Leder, Kunststoff, Papier, Pappe und Holz eingesetzt. Hierbei handelt es sich um Polymerketten, die durch Urethan- oder Harnstoffbindungen verbunden sind und ebenfalls Säure-Gruppen, wie zum Beispiel Carboxylate oder Sulfonate, oder alkalische Gruppen, wie zum Beispiel Amine, enthalten. Durch anschließende Neutralisation gelingt es, solche Polyurethane in stabile, wässrige Dispersionen zu überführen. Oftmals werden solche 1-K Systeme durch Zugabe eines wasserlöslichen Vernetzers, wie zum Beispiel wasserlösliche Melaminharze, nachvernetzt. Darüber hinaus können zusätzliche Gruppen im Molekül eingebaut sein, die nach Trocknung eine Vernetzung bewirken. Ein ausführliche Beschreibung von Polyurethandispersionen, deren chemisch-technologische Grundlagen und Anwendungsgebiete findet sich zum Beispiel im Buch "Polyurethane für Lacke und Beschichtungen" (Manfred Bock, Vincentz Verlag, Hannover 1999).

Es ist bekannt dass Lacke auf Basis von solchen Polyurethandispersionen ein oder mehrere Silicone oder organisch modifizierte Siloxane enthalten können, die die Anschmutzneigung und die Gleitreibung reduzieren, die Haftgleitreibung beziehungsweise Stick-Slip eliminieren, die Oberfläche hydrophobieren und die Haptik verbessern. Offenbart wird dies zum Beispiel in den Schriften DE 3839937 und WO 03/106575 Weiter Mikroemulsionen enthaltend Polysiloxane mit Ammoniumgruppen sind aus EP 1426398 A1, US 2007/0212326 A1 und WO 2011/042409 A2 bekannt

Nachteilig aller im Stand der Technik beschriebenen Mikroemulsionen ist es, dass alle als geeignet bezeichneten, nichtionischen Tenside ausnahmslos alkoxylierte Verbindungen darstellen.

Darüber hinaus enthalten alle im Stand der Technik beschriebenen Mikroemulsionen ausschließlich Siloxane, welche keinen ausgeprägten Siliconcharakter besitzen und keine gute Wirksamkeit als Konditioniermittel für z.B. Haare oder Textilien aufweisen, oder über eine geringe Affinität zu bestimmten Oberflächen verhältnismäßig verfügen. Aufgabe der vorliegenden Erfindung war es, eine Möglichkeit zu finden, Polysiloxane, insbesondere solche, die bei Raumtemperatur eine hohe Viskosität aufweisen, für Applikationsanwendungen handhabbar zu machen.

### Beschreibung der Erfindung

Überraschenderweise wurde gefunden, dass die im Folgenden beschriebenen Mikroemulsionen die der Erfindung gestellte Aufgabe zu lösen vermögen. Gegenstand der vorliegenden Erfindung sind daher Mikroemulsionen, welche als maßgeblich die Öl-Phase bildende Komponente bestimmte quaternäre Polysiloxane mit (Multi-)T-Struktur enthalten.

Ein weiterer Gegenstand der Erfindung ist ein Verfahren zur Herstellung der erfindungsgemäßen Mikroemulsionen, die Verwendung der erfindungsgemäßen Mikroemulsionen zur Herstellung von Formulierungen sowie die Formulierungen enthaltend die erfindungsgemäßen Mikroemulsionen.

Ein Vorteil der erfindungsgemäßen Mikroemulsionen ist es, dass hochviskose quaternäre Polysiloxane mit (Multi-)T-Struktur in einen niedrigviskosen Zustand überführt werden können, ohne ihre Funktionalität bzw. Aktivität zu verlieren, sondern im Gegenteil sogar die Aktivität in Anwendungen zu steigern.

Ein weiterer Vorteil der vorliegenden Erfindung ist, dass die quaternären Polysiloxane mit (Multi-)T-Struktur in Formulierungen eine bessere Performance erreichen. Die Mikroemulsionen der vorliegenden Erfindung verstärken die Konditionierungseigenschaften der enthaltenen quaternären Polysiloxane mit (Multi-)T-Struktur wie Kämmbarkeit, Weichheit, Volumen, Formbarkeit, Handhabbarkeit, die Entwirrbarkeit von ungeschädigten und geschädigten Haaren und Glanzeffekt sowie die Pflege- und Reinigungswirkung von Formulierungen für Haushalt und Industrie. Weiterhin wird durch den Einsatz von Mikroemulsionen ein verbessertes Weichgriffgefühl bei Textilien, insbesondere bei Textilien aus Baumwollstoffen. erzeugt, als bei der Verwendung von z.B. Makroemulsionen.

Ein weiterer Vorteil der vorliegenden Erfindung ist, dass quaternäre Polysiloxane mit (Multi-)T-Struktur in kosmetische Formulierungen eingearbeitet werden können, die in reiner Form bzw. verdünnt in geeigneten Lösungsmitteln auf Grund der hohen Viskosität oder der mangelnden Verträglichkeit nicht in kosmetische Formulierungen eingearbeitet werden können.

Noch ein Vorteil ist, dass die erfindungsgemäßen Mikroemulsionen für kosmetische Anwendungen im Wesentlichen frei von alkoxylierten Bestandteilen herstellbar sind. Noch ein Vorteil der erfindungsgemäßen Mikroemulsionen enthaltend Siliconquats mit (Multi-)T-Struktur ist, dass sie einen besonders guten konditionierenden Effekt in kosmetischen, dermatologischen und pharmazeutischen Formulierungen aufweisen. Ein weiterer Vorteil der Erfindung ist es, dass sonst nicht wasserlösliche quaternäre Polysiloxane mit (Multi-)T-Struktur in eine wasserverdünnbare Form überführt werden können.

Gegenstand der vorliegenden Erfindung ist eine Mikroemulsion als maßgeblich die Öl-Phase bildende Komponente enthaltend
A) mindestens eine quaternäre Ammoniumgruppe enthaltendes Polysiloxan der allgemeinen Formel (I)

   Mₐ M'ₐ₁ M"ₐ₂ M"'ₐ₃ D_{b} D'_{b1} D"_{b2} D'"_{b3} T_{c} T'_{c1} Q_{d} Formel (I),

   wobei
   M = (R¹₃SiO_{1/2})
   M' = (R²R¹₂SiO_{1/2})
   M" = (R³R¹₂SiO_{1/2})
   M'" = (R⁴R¹₂SiO_{1/2})
   D = (R¹₂SiO_{2/2})
   D' = (R²R¹SiO_{2/2})
   D" = (R³R¹SiO_{2/2})
   D'" = (R⁴R¹SiO_{2/2})
   T = (R⁵SiO_{3/2})
   T' = (R²SiO_{3/2})
   Q = (SiO_{4/2})
   a = 0 bis 32; bevorzugt 0 bis 22, insbesondere 0 bis 12;
   a1 = 0 bis 10, bevorzugt 0 bis 5, insbesondere 0;
   a2 = 0 bis 32; bevorzugt 0 bis 22, insbesondere 1 bis 12;
   a3 = 0 bis 10; bevorzugt 0 bis 5, insbesondere 0;
   mit der Maßgabe, dass
   a + a1 + a2 + a3 ≥ 3, insbesondere 3 bis 22, bevorzugt > 3, insbesondere 4 bis 17;
   b = 20 bis 400;
   b1 = 0 bis 10, bevorzugt 0 bis 5, insbesondere 0;
   b2 = 0 bis 80, bevorzugt 0 bis 50, insbesondere 0 bis 10;
   b3 = 0 bis 20, bevorzugt 0 bis 10, insbesondere 0;
   c = 0 bis 30, bevorzugt 1 bis 20, insbesondere 2 bis 15;
   c1 = 0 bis 10, bevorzugt 0 bis 5, insbesondere 0;
   d = 0 bis 15, bevorzugt 1 bis 12, insbesondere 2 bis 10;
   mit der Maßgabe, dass
   a2 + b2 ≥ 1, bevorzugt ≥ 3, insbesondere >3 und
   c + c1 + d ≥ 1, bevorzugt 1 bis 20, bevorzugt > 1, insbesondere 2 bis 15, insbesondere ≥ 3;
   R¹ = Methyl;
   R² = unabhängig voneinander gleiche oder verschiedene Alkoxy- oder Acyloxyreste, wie beispielsweise Methoxy-, Ethoxy-, n-Propoxy- oder iso-Propoxyreste, Acetoxy, insbesondere Ethoxy- oder iso-Propoxyreste;
   R³ = unabhängig voneinander gleiche oder verschiedene organische Reste, die quaternäre Ammoniumfunktionen tragen;
   R⁴ = unabhängig voneinander gleiche oder verschiedene organische Epoxy-Reste;
   R⁵ = unabhängig voneinander gleiche oder verschiedene Reste R¹, R³ oder R⁴, bevorzugt R¹, insbesondere Methyl, Phenyl, Dodecyl oder Hexadecyl
   sind.

Unter dem Begriff "maßgeblich die Öl-Phase bildende Komponente" wird im Zusammenhang mit der vorliegenden Erfindung verstanden, dass das Polysiloxan mindestens 50 Gew.-% der Öl-Phase ausmacht; der restliche Anteil der Öl-Phase kann beispielsweise aus der im Folgenden als Komponente G bezeichneten Komponete bestehen.

Die im Rahmen der Erfindung beschriebenen Polysiloxane können verschiedene Einheiten mehrfach aufweisen, diese können statistisch verteilt (statistisches Oligomer) oder geordnet (Blockoligomer) in diesen Verbindungen vorkommen. Es ist dem Fachmann geläufig, dass die Verbindungen in Form eines Gemisches mit einer im Wesentlichen durch statistische Gesetze geregelten Verteilung vorliegen. Angaben zu Anzahl von Einheiten in solchen Verbindungen sind als Mittelwert, gemittelt über alle entsprechenden Verbindungen zu verstehen. Alle angegebenen Prozent (%) sind, wenn nicht anders angegeben, Massenprozent. Alle Bedingungen wie beispielsweise Druck und Temperatur sind, wenn nicht anders angegeben, Standardbedingungen.

In den erfindungsgemäßen Mikroemulsionen enthaltene Polysiloxane enthalten bevorzugt als Epoxy-Reste R⁴, vorzugsweise gleiche oder verschiedene Reste ausgewählt aus der Gruppe 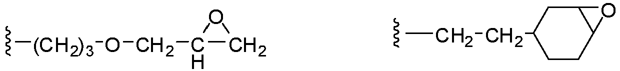 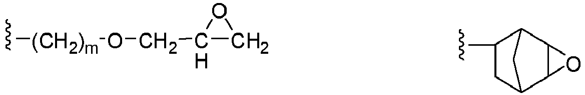 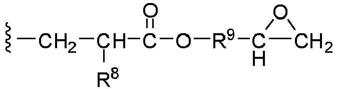 Geeignete Reste R³ sind zum Beispiel Gruppen mit dem Aufbau

-R⁶-R⁷,

worin
- R⁶: vorzugsweise gleiche oder verschiedene zweiwertige Reste sind, ausgewählt aus der Gruppe 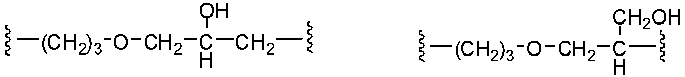 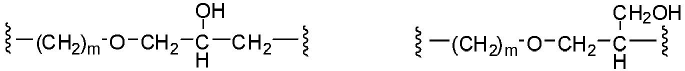 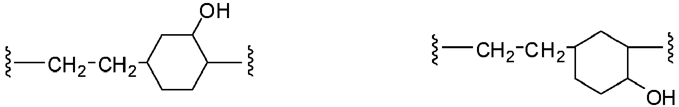 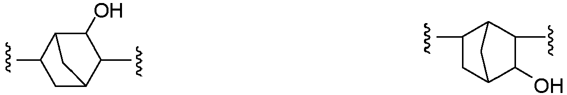 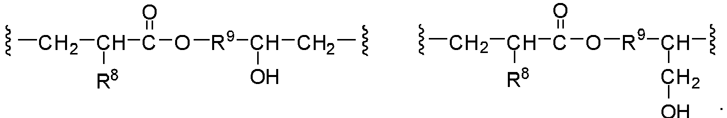
- R⁶: ist besonders bevorzugt: 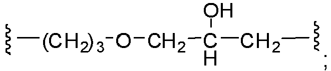
- R⁷: ausgewählt ist aus der Gruppe bestehend aus 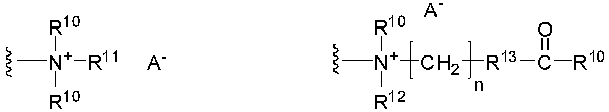 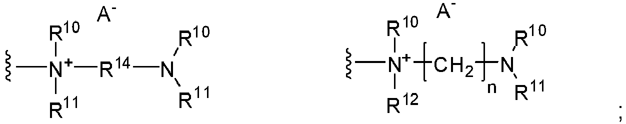
- R⁸: sind gleiche oder verschiedene Reste aus der Gruppe Wasserstoff oder Alkyl mit 1 bis 6 C-Atomen, bevorzugt Methyl;
- R⁹: sind gleiche oder verschiedene zweiwertige Kohlenwasserstoffreste, die gegebenenfalls Etherfunktionen enthalten, bevorzugt Methylen;
- R¹⁰, R¹¹, R¹²: sind jeweils unabhängig voneinander Wasserstoff oder Alkylreste mit 1 bis 30 C-Atomen oder Reste der Formel 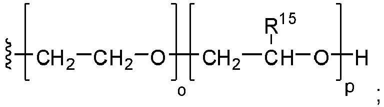
- R¹³: sind gleiche oder verschiedene Reste aus der Gruppe

-O-, -NR¹⁶-;
- R¹⁴: sind gleiche oder verschiedene gegebenenfalls verzweigte divalente Kohlenwasserstoffreste, bevorzugt Ethylen oder Propylen;
- R¹⁵: sind gleiche oder verschiedene Alkyl-, Aryl- oder Alkarylreste mit 1 bis 30 C-Atomen, die gegebenenfalls Etherfunktionen enthalten, bevorzugt Methyl, Ethyl oder Phenyl, insbesondere Methyl;
- R¹⁶: sind gleiche oder verschiedene Reste aus der Gruppe Wasserstoff oder Alkyl mit 1 bis 6 C-Atomen;
- m: = 2 bis 18;
- n: = 2 bis 18, bevorzugt 3;
- o: = 0 bis 30, bevorzugt 0 bis 10, insbesondere 1 bis 3;
- p: = 0 bis 30, bevorzugt 0 bis 10;
A⁻ sind gleiche oder verschiedene Gegenionen zu den positiven Ladungen an den quaternierten Stickstoffgruppen, ausgewählt aus anorganischen oder organischen Anionen der Säuren HA, sowie deren Derivate.

In einer weiteren bevorzugten Ausführungsform der vorliegenden Erfindung besteht das Gegenion A⁻ zu den positiven Ladungen an den quaternierten Stickstoffgruppen aus dem Anion einer physiologisch verträglichen Säure HA, die besonders bevorzugt aus Essigsäure, L-Hydroxycarbonsäuren, insbesondere Milchsäure, oder aromatischen Carbonsäuren ausgewählt ist.

Weitere bevorzugte Gegenionen stammen aus gängigen Quaternierungsmitteln. Dies sind insbesondere Ethylsulfat, Methylsulfat, Toluolsulfonat, Chlorid und Bromid.

Es ist eine Ausgestaltungsform der vorliegenden Erfindung, enthalten die erfindungsgemäßen Mikroemulsionen mindestens ein Polysiloxan, welches eine einfache T-Struktur mit endständiger Modifizierung aufweist, somit dadurch gekennzeichnet ist, dass die Mikroemlusion ein Polysiloxan enthält mit c = 1 und c + c1 + d = 1 (folglich c1 = d = 0) sowie a2 ≥ 1 mit a2 + a3 = 3 und a = a1 = b1 = b2 = b3 = 0. Die in den erfindungsgemäßen Mikroemulsionen enthaltenden Polysiloxane lassen sich nach Verfahren, wie in den bereits oben genannten Schriften EP 1887024 und WO2011/088937 A1 beschrieben, herstellen, wobei für die Herstellung der Polysiloxane mit einfacher T-Struktur bevorzugt das in der EP 1887024 genannte Verfahren eingesetzt wird. Es wird bezüglich der Herstellungsverfahren explizit auf die Offenbarung dieser Schriften hingewiesen, welche die Beschreibung in der vorliegenden Schrift ersetzt.

In den Mikroemulsionen der vorliegenden Erfindung werden vorteilhaft Polysiloxane mit einem mittleren Molekulargewicht von größer 4000 g/mol, bevorzugt von größer 7000 g/mol, insbesondere von größer 10000 g/mol eingesetzt, wobei das mittlere Molekulargewicht mittels ²⁹Si-NMR bestimmt wird.

Erfindungsgemäß bevorzugte Mikroemulsionen weisen eine Domänengröße der dispersen Phase von kleiner 1000 nm, insbesondere kleiner 500 nm auf, wobei die Bestimmung der Domänengröße mit Hilfe dem Fachmann bekannter Streumethoden durchgeführt wird, wie beispielsweise in P. Lindner und Th. Zemb, "Neutrons, X-Rays and Light: Scattering Methods Applied to Soft Condensed Matter", Elsevier Science & Technology, November 2002 oder O. Glatter und O. Kratky, "Small-angle X-ray Scattering" Academic Press Inc, Dezember 1982, beschrieben.

In den Mikroemulsionen der vorliegenden Erfindung werden vorteilhaft Polysiloxane mit einer Viskosität von > 1 Pas, insbesondere von 10 Pas bis 100.000 Pas, (gemessen mit einer Platte-Platte-Geometrie eines Rheometers bei T =25 °C, Spaltbreite von 1 mm bei einer Scherrate von 1 s⁻¹) eingesetzt.

Erfindungsgemäß bevorzugte Mikroemulsionen sind dadurch gekennzeichnet, dass sie zusätzlich mindestens eine der Komponenten enthalten
B) mindestens ein nichtionisches Tensid;
C) ein Cotensid ausgewählt aus der Gruppe enthaltend, bevorzugt bestehend aus anionischen, kationischen und amphoteren Tensiden; und
D) Wasser.

Erfindungsgemäß bevorzugte Mikroemulsionen sind dadurch gekennzeichnet, dass sie zusätzlich mindestens eine der Komponenten B) und C) oder B) und D) oder C) und D) oder B) und C) und D) enthalten.

Bevorzugte nichtionische Tenside sind ausgewählt aus der Gruppe enthaltend, bevorzugt bestehend aus,
Anlagerungsprodukten von Ethylenoxid und/oder Propylenoxid an lineare Fettalkohole, Fettsäuren, Fettsäureamide, Fettamine und an Alkylphenole,
Glycerinmono- und -diester und Sorbitanmono- und -diester von gesättigten und ungesättigten Fettsäuren und deren Ethylenoxidanlagerungsprodukte,
Alkylmono- und -oligoglycoside und deren Ethylenoxidanlagerungsprodukte, Anlagerungsprodukte von Ethylenoxid an Rizinusöl und/oder gehärtetes Rizinusöl, Partialester auf Basis linearer, verzweigter, ungesättigter bzw. gesättigter Fettsäuren, Ricinolsäure, 12-Hydroxystearinsäure, Glycerin, Polyglycerin, Pentaerythrit, Dipentaerythrit und Zuckeralkohole (z.B. Sorbit), Alkylglucoside (z.B. Methylglucosid, Butylglucosid, Laurylglucosid) sowie Polyglucoside (z.B. Cellulose), Mono-, Di- und Trialkylphosphate sowie Mono-, Di- und/oder Tri-PEG-alkylphosphate und deren Salze, Zitronensäureester wie z.B. Glyceryl Stearate Citrate, Glyceryl Oleate Citrate und Dilauryl Citrate sowie Glyceryl Caprylate, Polyglycerylcaprylate, Polyglycerylcaprate, weiter alkoxylierte Triglyceride, Mischether bzw. Mischformale, gegebenenfalls partiell oxidierte Alkyloligoglykoside oder Alkenyloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Polysorbate und Aminoxide
und Mischungen dieser Tenside.

Sofern die nichtionischen Tenside Polyglycoletherketten enthalten, können diese eine konventionelle, vorzugsweise jedoch eine eingeengte Homologenverteilung aufweisen.

Im Zusammenhang mit der vorliegenden Erfindung sind unter dem Begriff "Fettsäuren" insbesondere Ameisensäure, Essigsäure, Propionsäure, Buttersäure, Valeriansäure, Capronsäure, Önanthsäure, Caprylsäure, Pelargonsäure, Caprinsäure, Laurinsäure, Myristinsäure, Pentadecansäure, Palmitinsäure, Margarinsäure, Stearinsäure, Nonadecansäure, Arachinsäure, Behensäure, Lignocerinsäure, Cerotinsäure, Montansäure, Melissinsäure, Undecylensäure, Myristoleinsäure, Palmitoleinsäure, Petroselinsäure, Ölsäure, Elaidinsäure, Vaccensäure, Gadoleinsäure, Icosensäure, Cetoleinsäure, Erucasäure, Nervonsäure, Linolsäure, Alpha-Linolensäure, Gamma-Linolensäure, Calendulasäure, Punicinsäure, Alpha-Elaeostearinsäure, Beta-Elaeostearinsäure, Arachidonsäure, Timnodonsäure, Clupanodonsäure, Cervonsäure, Vernolsäure, Rizinolsäure zu verstehen, wobei solche mit einer Kettenlänge von 6 bis 22, insbesondere 8 bis 18 C-Atomen besonders bevorzugt sind.; analoges gilt für das Kohlenstoffgrundgerüst für den im Zusammenhang mit der Erfindung verwendeten Begriff "Fettalkohole".

Bevorzugte anionische Tenside sind solche, aufweisend eine Carboxylat-, Sulfat-, Sulfonat- oder Phosphat-Gruppe und einen lipophilen Rest.
Typische Beispiele für anionische Tenside sind Seifen, Alkylbenzolsulfonate, Alkansulfonate, Olefinsulfonate, Alkylethersulfonate, Glycerinethersulfonate, alpha - Methylestersulfonate, Sulfofettsäuren, Alkylsulfate, Alkylethersulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Alkylethercarboxylate, Ethercarbonsäuren und deren Salze, Acylsarcosinate Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate und Mischungen dieser Tenside.
Sofern die anionischen Tenside Polyglycoletherketten enthalten, können diese eine konventionelle, vorzugsweise jedoch eine eingeengte Homologenverteilung aufweisen.

Als kationische Tenside können insbesondere quaternäre Ammoniumverbindungen, insbesondere solche, versehen mit mindestens einer linearen und/oder verzweigten, gesättigten oder ungesättigten Alkylkette, eingesetzt werden, so etwa Alkyltrimethylammoniumhalogenide wie z.B. Cetyltrimethylammoniumchlorid oderbromid oder Behenyltrimethylammoniumchlorid, aber auch Dialkyldimethylammoniumhalogenide wie z.B. Distearyldimethylammoniumchlorid. Weiterhin können als kationische Tenside Monoalklyamidoquats wie z.B. Palmitamidopropyltrimethylammoniumchlorid oder entsprechende Dialkylamidoquats eingesetzt werden.
Weiterhin können als kationische Tenside quaternäre Esterverbindungen eingesetzt werden, bei denen es sich um quaternierte Fettsäureester auf Basis von Mono-, Di- oder Triethanolamin handeln kann. Kationische Tenside können weiterhin Alkylguanidiniumsalze sein.

Typische Beispiele für amphotere Tenside sind Amphoacetate, Amphopropionate, Alkylbetaine, Alkylamidobetaine, Aminopropionate, Aminoglycinate, Imidazoliniumbetaine und Sulfobetaine wie z.B. die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Insbesondere für kosmetische oder topische Anwendungen sind Mikroemulsionen bevorzugt, die im Wesentlichen frei von alkoxylierten Verbindungen sind. Unter dem Begriff "im Wesentlichen frei von alkoxylierten Verbindungen" im Zusammenhang mit der vorliegenden Erfindung ist zu verstehen, dass die Mikroemulsion keine nennenswerten Mengen an alkoxylierten Verbindungen aufweist, die eine oberflächenaktive Wirkung ausüben. Insbesondere ist hierunter zu verstehen, dass alkoxylierte Verbindungen in Mengen von kleiner 1 Gew.-%, bevorzugt von kleiner 0,1 Gew.-%, besonders bevorzugt von kleiner 0,01 Gew.-% bezogen auf die Gesamtmikroemulsion, insbesondere keine nachweisbaren Mengen enthalten sind. In solchen, im Wesentlichen von alkoxylierten Verbindungen freien Mikroemulsionen sind insbesondere nichtionische Tenside der Komponente B) ausgewählt aus der Gruppe bestehend aus
Glycerinmono- und -diester und Sorbitanmono- und -diester von gesättigten und ungesättigten Fettsäuren,
Alkylmono- und -oligoglycoside, Partialester auf Basis linearer, verzweigter, ungesättigter bzw. gesättigter Fettsäuren,
Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose),
Mono-, Di- und Trialkylphosphate und deren Salze,
Zitronensäureester wie z.B. Glyceryl Stearate Citrate, Glyceryl Oleate Citrate und Dilauryl Citrate sowie Glyceryl Caprylate, Polyglycerylcaprylate, Polyglycerylcaprate und Mischungen dieser Tenside enthalten.

Da im Herstellungsverfahren der Mikroemulsionen die Anwesenheit eines Lösungsmittels die Herstellung der Emulsion vereinfachen kann, sind bevorzugte Mikroemulsionen der vorliegenden Erfindung dadurch gekennzeichnet, dass zusätzlich ein Lösungsmittel E) enthalten ist ausgewählt aus der Gruppe enthaltend, bevorzugt bestehend aus,
Hydrotropen beispielsweise aus der Gruppe der aliphatischen Alkohole, wie Ethanol, Propanol oder 1,3-Propandiol, zyklische Carbonate wie Ethylencarbonat, Propylencarbonat, Glycerincarbonat, Ester von Mono- oder Polycarbonsäuren wie Ethylacetat, Ethyllactat, Glycerin, Isopropylalkohol, Dipropylenglykol, Glykolether (beispielsweise unter dem Namen DOWANOL® von Dow Chemicals erhältlich) oder Polyole. Polyole, die hier in Betracht kommen, können 2 bis 15 Kohlenstoffatome und mindestens zwei Hydroxylgruppen besitzen. Typische Beispiele sind: Glycerin, Alkylenglycole, wie beispielsweise Ethylenglycol, Diethylenglycol, Propylenglycol, Butylenglycol, Pentylenglycol, Hexylenglycol, 1,2-Propandiol, 1,2-Butandiol, 1,2-Pentandiol, 1,2-Hexandiol sowie Polyethylenglycol oder Polypropylenglycol, Polyhydroxycarbonsäuren, Butyldiglykol und Gemische dieser Lösungsmittel.

Um die erfindungsgemäßen Mikroemulsionen mikrobiologisch zu stabilisieren, ist es vorteilhaft, wenn diese eine Komponente F), Konservierungsmittel, enthalten. Solche können beispielsweise Mischungen einzelner oder mehrerer Alkylparabenester mit Phenoxyethanol alleine sein. Bei den Alkylparabenestern kann es sich um Methlyparaben, Ethylparaben, Propylparaben und/oder Butylparaben handeln. Anstelle von Phenoxyethanol können auch andere Alkohole eingesetzt werden, wie beispielsweise Benzylalkohol oder Ethanol. Darüber hinaus können auch andere Konservierungsmittel, alleine oder in Mischung, wie etwa Phenoxyethanol, Sorbin- oder Benzoesäure, Salicylsäure, 2-Bromo-2-Nitropropan-1,3-Diol, Chloracetamid, Diazolidinyl Harnstoff, DMDM Hydantoin, lodopropynyl Butylcarbamat, Natrium Hydroxymethylglycinate, Methylisothiazolin, Chlormethyl-isothiazolin, Ethylhexylglycerin oder Caprylyl Glycol eingesetzt werden.

Die Öl-Phase der erfindungsgemäßen Mikroemulsion kann zusätzlich eine Komponente G), ein Öl oder Ölgemisch, ausgewählt aus der Gruppe enthaltend, bevorzugt bestehend aus, Propylene Glycol Monocaprylaten, Mono- oder Diestern von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen; sowie Veresterungsprodukte aliphatischer, difunktioneller Alkohole mit 2 bis 36 C-Atomen mit monofunktionellen aliphatischen Carbonsäuren mit 1 bis 22 C-Atomen, langkettige Arylsäureester von linearen oder verzweigten, gesättigten oder ungesättigten Alkoholen mit 1 bis 22 C-Atomen, oder auch Benzoesäureisostearylester oder Benzoesäureoctyldocecylester, Monoester (wie z. B. die Methylester und Isopropylester von Fettsäuren mit 12 bis 22 C-Atomen wie z. B. Methyllaurat, Methylstearat, Methyloleat, Methylerucat, Isopropylpalmitat, Isopropylmyristat, Isopropylstearat, Isopropyloleat, n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat) sowie Ester, die aus technischen aliphatischen Alkoholschnitten und technischen, aliphatischen Carbonsäuregemischen erhältlich sind; sowie natürlich vorkommende Monoester- bzw. Wachsester-Gemische, wie sie z. B. im Jojobaöl oder im Spermöl vorliegen. Geeignete Dicarbonsäureester sind z. B. Di-n-butyl-adipat, Di-n-butyl-sebacat, Di-(2-ethylhexyl)-adipat, Di-(2-hexyldecyl)-succinat, D-isotridecylacelaat. Geeignete Diolester sind z. B. Ethylenglycoldioleat, Ethylenglycol-di-isotridecanoat, Propylenglycol-di-(2-ethylhexanoat), Butandiol-di-isostearat, Butandiol-di-caprylat/caprat und Neopentylglycol-di-caprylat. Weitere Fettsäureester, die eingesetzt werden können, sind z. B. C12-15 Alkylbenzoat, Dicaprylylcarbonat, Diethylhexylcarbonat. Ebenso als Ölkomponente können längerkettige Triglyceride, d. h. dreifache Ester des Glycerins mit drei Säuremolekülen, wovon mindestens eine längerkettig ist, eingesetzt werden; weiterhin Kohlenwasserstoffe, insbesondere auch flüssige Paraffine und Isoparaffine wie Paraffinöl, Isohexadecan, Polydecen, Vaseline, Paraffinum perliquidum, Squalan, Ceresin; sowie lineare oder verzweigte Fettalkohole wie Oleylalkohol oder Octyldodecanol und Fettalkoholether wie Dicaprylyl Ether; Siliconöle und -wachse wie z. B. Polydimethylsiloxane, Cyclomethylsiloxane, sowie aryl- oder alkyl- oder alkoxy-substituierte Polymethylsiloxane oder Cyclomethylsiloxane, Guerbetalkohole auf Basis von Fettalkoholen mit 6 bis 18, vorzugsweise 8 bis 10 Kohlenstoffatomen, Ester von linearen C6-C22-Fettsäuren mit linearen C6-C22-Fettalkoholen, Ester von verzweigten C6-C13-Carbonsäuren mit linearen C6-C22-Fettalkoholen, Ester von linearen C6-C22-Fettsäuren mit verzweigten C8-C18-Alkoholen, insbesondere 2-Ethylhexanol oder Isononanol, Ester von verzweigten C6-C13-Carbonsäuren mit verzweigten Alkoholen, insbesondere 2-Ethylhexanol oder Isononanol, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C6-C10-Fettsäuren, flüssige Mono-/Di-/Triglyceridmischungen auf Basis von C6-C18-Fettsäuren, Ester von C6-C22-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C6-C22-Fettalkoholcarbonate, Guerbetcarbonate, Ester der Benzoesäure mit linearen und/oder verzweigten C6-C22-Alkoholen (z. B. Finsolv™ TN), Dialkylether, Ringöffnungsprodukte von epoxidierten Fettsäureestern mit Polyolen, Siliconöle und/oder aliphatische bzw. naphthenische Kohlenwasserstoffe enthalten.
In diesem Zusammenhang gilt die Maßgabe, dass die Komponente G) maximal 50 Gew.-% der gesamten Ölphase ausmacht.

In erfindungsgemäß bevorzugten Mikroemulsionen ist Komponente A) in einer Menge von 10 Gew.-% bis 60 Gew.-%, bevorzugt in einer Menge von 15 Gew.-% bis 50 Gew.-%, besonders bevorzugt in einer Menge von 20 Gew.-% bis 45 Gew.-% enthalten, wobei sich die Gew.-% auf die Gesamtmikroemulsion beziehen.

Besonders bevorzugte Mikroemulsion der vorliegenden Erfindung sind dadurch gekennzeichnet, dass
Komponente A) in einer Menge von 10 Gew.-% bis 60 Gew.-%, bevorzugt in einer Menge von 15 Gew.-% bis 50 Gew.-%, besonders bevorzugt in einer Menge von 20 Gew.-% bis 45 Gew.-%,
Komponente B) in einer Menge von 3 Gew.-% bis 30 Gew.-%, bevorzugt in einer Menge von 4 Gew.-% bis 20 Gew.-%, besonders bevorzugt in einer Menge von 5 Gew.-% bis 15 Gew.-%,
Komponente C) in einer Menge von 0 Gew.-% bis 30 Gew.-%, bevorzugt in einer Menge von 3 Gew.-% bis 25 Gew.-%, besonders bevorzugt in einer Menge von 5 Gew.-% bis 20 Gew.-%,
Komponente D) in einer Menge von 10 Gew.-% bis 75 Gew.-%, bevorzugt in einer Menge von 15 Gew.-% bis 65 Gew.-%, besonders bevorzugt in einer Menge von 20 Gew.-% bis 55 Gew.-%,
Komponente E) in einer Menge von 0 Gew.-% bis 35 Gew.-%, bevorzugt in einer Menge von 3 Gew.-% bis 30 Gew.-%, besonders bevorzugt in einer Menge von 5 Gew.-% bis 25 Gew.-%,
Komponente F) in einer Menge von 0 Gew.-% bis 1 Gew.-%, bevorzugt in einer Menge von 0,0001 Gew.-% bis 0,5 Gew.-%, und
Komponente G) in einer Menge von 0 Gew.-% bis 50 Gew.-%, bevorzugt in einer Menge von 1 Gew.-% bis 40 Gew.-%, besonders bevorzugt in einer Menge von 5 Gew.-% bis 20 Gew.-% der gesamten Öl-Phase bestehend aus A) und G),
enthalten ist, wobei sich die Gew.-% außer im Falle von Komponente G) auf die Gesamtmikroemulsion beziehen.

Ein weiterer Gegenstand der vorliegenden Erfindung ist ein Verfahren zur Herstellung einer erfindungsgemäßen Mikroemulsion umfassend die Verfahrensschritte
I) Bereitstellen mindestens eines Polysiloxans wie oben beschrieben,
II) gegebenenfalls Lösen des Polysiloxans mit mindestens einem Lösungsmittel E) und/oder einem nichtionischen Tensid der Komponente B) und/oder Öl G)
III) hinzugeben der restlichen die Mikroemulsion bildenden Komponenten, wobei die Komponenten Wasser und Konservierungsmittel zuletzt zugegeben werden.

Die in dem erfindungsgemäßen Verfahrenen beschriebenen Komponenten A) bis G) entsprechen den oben im Zusammenhang mit den erfindungsgemäßen Mikroemulsionen beschriebenen Komponenten, wobei jeweils bevorzugte Komponenten der Mikroemulsionen selbstverständlich in dem erfindungsgemäßen Verfahren bevorzugt eingesetzt werden; der Vollständigkeit halber sei angeführt, dass diese Aussage natürlich für die eingesetzten Mengen der einzelnen Komponenten gilt.

In dem erfindungsgemäßen Verfahren müssen keine starken mechanischen Kräfte angewandt werden, um eine Emulsion auszubilden, da Mikroemulsionen sich spontan ausbildende Emulsionen sind; dennoch ist es erfindungsgemäß bevorzugt dass während aller Verfahrensschritte I) - III) mit einem einfachen Rührer, wie beispielsweise einem Pendelrührer, gemischt wird.

In erfindungsgemäß bevorzugten Verfahren werden oben im Zusammenhang mit den erfindungsgemäßen Mikroemulsionen als bevorzugt beschriebene Polysiloxane bevorzugt eingesetzt.

Um während der Herstellung der Mikroemulsion eventuell auftretende hochviskose Zustände zu umgehen, kann es von Vorteil sein, wenn das Verfahren gemäß der vorliegenden Erfindung dadurch gekennzeichnet ist, dass Verfahrensschritte I) - III), bei leicht erhöhter Temperatur, beispielsweise in einem Temperaturbereich von 21 °C bis 90 °C, insbesondere von 30 °C bis 50 °C, durchgeführt werden.

Die Mikroemulsionen der vorliegenden Erfindung lassen sich vorteilhaft zur Herstellung von Pflege- und Reinigungsformulierungen, insbesondere für Haut und Hautanhangsgebilden, wie beispielsweise Konditioniermittel für Haare, sowie von Pflege- und Reinigungsformulierungen für Haushalt und Industrie verwenden, wobei diese bevorzugt ausgewählt sind aus der Gruppe der kosmetischen, reinigenden und pflegenden Formulierungen. Somit sind derartige Verwendungen ebenfalls Gegenstand der vorliegenden Erfindung.
Unter dem Begriff "Pflegeformulierung" wird hier eine Formulierung verstanden, die den Zweck erfüllt, einen Gegenstand in seiner ursprünglichen Form zu erhalten, die Auswirkungen äußerer Einflüsse (z.B. Zeit, Licht, Temperatur, Druck, Verschmutzung, chemische Reaktion mit anderen, mit dem Gegenstand in Kontakt tretenden reaktiven Verbindungen) wie beispielsweise Altern, Verschmutzen, Materialermüdung, Ausbleichen, zu mindern oder zu vermeiden oder sogar gewünschte positive Eigenschaften des Gegenstandes zu verbessern. Für letzten Punkt sei etwa ein verbesserter Haarglanz oder eine größere Elastizität des betrachteten Gegenstandes genannt.
In diesem Zusammenhang sind die Pflege- und Reinigungsformulierungen nicht auf kosmetische, pharmazeutische oder dermatologische Formulierungen wie z. B. zur Behandlung der Haare in Form von Haarshampoos, 2in1-Shampoos, Flüssigseifen, Haarspülungen, Dauerwell-Fixierlösungen, Haarfärbeshampoos, Haarfestiger, Haarkuren, Haarlegemittel, Haarstyling-Zubereitungen, Fön-Lotionen, Schaumfestiger, Haarkuren, Leave-In Conditionierer, Haarglättungsmitteln, Glanzverbesserungsmitteln und Mittel zum Färben der Haare sowie anderen reinigenden und pflegenden Formulierungen beschränkt, sondern können auch solche Formulierungen sein, wie man sie in Haushalt und Industrie einsetzt, etwa zur Pflege und Reinigung von Oberflächen nicht lebender Gegenstände wie beispielsweise Fliesen, Holz, Glas, Keramik, Linoleum, Kunststoff, lackierte Oberflächen, Leder, Stoffe, Fasern. Beispiele solcher Gegenstände sind Fensterscheiben und -bänke, Duschabtrennungen, Fußböden wie Teppiche, Fliesen, Laminate, Parkett, Korkfußböden, Marmor-, Stein- und Feinsteinzeugböden, Haushaltskeramiken wie WCs, Waschbecken, Bidets, Duschtassen, Badewannen, Türklinken, Armaturen, Haushaltswerkzeuge wie Waschmaschinen, Trockner, Spülmaschinen, Spülen aus Keramik oder Edelstahl, Möbel wie Tische, Stühle, Regale, Ablageflächen, Fenster, Kochgeschirr, Geschirr und Besteck, Wäsche, insbesondere körpernahe Wäsche ("Unterwäsche"), Wasserfahr-, Fahr- und Flugzeuge wie Autos, Busse, Motor- und Segelboote Werkzeuge wie chirurgische Instrumente, Staubsauger, Maschinen, Rohrleitungen, Tanks und Geräte für Transport, Verarbeitung und Aufbewahrung in der Lebensmittelverarbeitung. Somit handelt es sich in diesem Zusammenhang um die Verwendung in Reinigungs- und Pflegemitteln für Haushalt, industrielle und institutionelle Gewerbe.
In diesem Zusammenhang handelt es sich bei der zu pflegenden und reinigenden Oberfläche bevorzugt um die einer Faser oder eines Textils, insbesondere um die Oberfläche von gewobenen Textilien, Wäsche, Polstern oder Teppichen.
Ein weiterer Gegenstand dieser Erfindung ist die Verwendung der erfindungsgemäßen Mikroemulsionen als Konditioniermittel für Haarbehandlungsmittel und Haarnachbehandlungsmittel sowie als Mittel zur Verbesserung der Haarstruktur.

Noch ein Gegenstand der vorliegenden Erfindung sind somit auch die Pflege- und Reinigungsformulierungen, insbesondere für Haut und Hautanhangsgebilden, sowie von Pflege- und Reinigungsformulierungen für Haushalt und Industrie, insbesondere kosmetische Formulierungen, wobei diese bevorzugt ausgewählt sind aus der Gruppe der Haarbehandlungsmittel und Haarnachbehandlungsmittel zum Ausspülen oder zum Verbleib im Haar, beispielsweise Shampoos mit oder ohne ausgeprägter Konditionierwirkung, 2in1-Shampoos, Spülungen, Haarkuren, Haarmasken, Frisierhilfen, Stylingmittel, Fönlotionen, Haarfestiger, Dauerwellmittel, Haarglättungsmittel und Mittel zum Färben der Haare, insbesondere Konditioniermittel und Shampoos enthaltend mindestens eine erfindungsgemäße Mikroemulsion. Erfindungsgemäß besonders bevorzugte kosmetische Formulierungen sind selber auch im Wesentlichen frei von alkoxylierten Verbindungen.
Bevorzugte erfindungsgemäße Reinigungs- und Pflegeformulierungen für Haushalt, industrielle und institutionelle Anwendungen enthaltend mindestens eine der erfindungsgemäßen Mikroemulsionen sind Desinfektionsmittel, Desinfektionsreiniger, Schaumreiniger, Fußbodenreiniger, Teppichreiniger, Polsterreiniger, Fußbodenpflegeprodukte, Marmorreiniger, Parkettreiniger, Stein- und Keramikbodenreiniger, Wischpflegemittel, Edelstahlreiniger, Glasreiniger, Geschirrspülmittel, Kunststoffreiniger, Sanitärreiniger, Holzreiniger, Lederreiniger, Waschmittel, Wäschepflegemittel Desinfektionswaschmittel, Vollwaschmittel, Feinwaschmittel, Wollwaschmittel, Weichspülmittel und Imprägniermittel, wobei Waschmittel, Wäschepflegemittel, Vollwaschmittel, Feinwaschmittel, Wollwaschmittel, Weichspülmittel, Imprägniermittel, insbesondere Weichspülmittel besonders bevorzugt sind.
Besonders bevorzugte erfindungsgemäße Reinigungs- und Pflegeformulierungen für Haushalt, industrielle und institutionelle Anwendungen enthaltend mindestens eine der erfindungsgemäßen Mikroemulsionen sind textilpflegenden Formulierungen für die wiederholte Reinigung und Pflege von Textilien und Geweben. Unter einer textilpflegenden Formulierung wird in diesem Zusammenhang jede Formulierung verstanden, die damit behandelten textilen Flächengebilden einen vorteilhaften Effekt vermittelt, wie beispielsweise einen textilweichmachenden Effekt, Knitterfestigkeit bzw. die schädliche oder negative Effekte, die beim Reinigen und/oder Konditionieren und/oder Tragen auftreten können, wie beispielsweise Verblassen, Vergrauung, usw., reduziert. Besonders bevorzugt ist, dass die textilpflegende Formulierung eine textilweichmachende Formulierung (Weichspüler) ist.
Textilweichmachende Formulierungen (Weichspüler) sind insbesondere wässrige (d.h. mit einem Gewichtsanteil von mindestens 5 Gew.-% Wasser bezogen auf die gesamte Formulierung) Formulierungen, die als Hauptwirkungsbestandteil eine oder mehrere kationische textilweichmachende Verbindung, die eine oder mehrere langkettige Alkylgruppen in einem Molekül aufweisen, enthalten. Weit verbreitete kationische textilweichmachende Verbindungen umfassen beispielsweise Methyl-N-(2-hydroxyethyl)-N,N-di(talgacyloxyethyl)ammonium-Verbindungen oder N,N-Dimethyl-N,N-di(talgacyloxyethyl)ammonium-Verbindungen. Weitere geeignete Ammoniumverbindungen offenbart die US 2010/0184634 in den Absätzen [0027] bis [0068], Die textilweichmachenden Formulierungen können darüber hinaus weitere Additive und Hilfsstoffe enthalten, insbesondere Parfüm, Farbstoffe, Viskositätsregulatoren, Entschäumer, Konservierungsmittel, organische Lösemittel, nicht siloxanhaltige Polymere und andere siloxanhaltige Polymere. Insbesondere können die textilweichmachenden Formulierungen zwischen 0,001 und 25, besonders bevorzugt 0,01 bis 15 Gew.-% eines oder mehrerer unterschiedlicher Additive oder Hilfsstoffe enthalten, wobei sich die Gew.-% auf die gesamte Formulierung beziehen.
Als Parfüm können alle für textilweichmachende Formulierungen aus dem Stand der Technik als geeignet bekannte Duftstoffe oder Duftstoffmischungen eingesetzt werden, vorzugsweise in Form eines Parfümöles. Beispiele für Duft- bzw. Riechstoffe werden unter anderem in der DE 197 51 151 A1, Seite 4, Zeile 11 - 17 offenbart. Insbesondere können die textilweichmachenden Formulierungen zwischen 0,01 und 10, besonders bevorzugt 0,1 bis 5 Gew.-% eines oder mehrerer Duftstoffe oder Duftstoffmischungen enthalten, wobei sich die Gew.-% auf die gesamte Formulierung beziehen.
Als Farbstoffe können alle für textilweichmachende Formulierungen aus dem Stand der Technik als geeignet bekannte Farbstoffe eingesetzt werden, wobei wasserlösliche Farbstoffe bevorzugt sind. Beispiele für geeignete wasserlösliche Farbstoffe sind SANDOLAN® Walkblau NBL 150 (Hersteller Clariant) und Sicovit® Azorubin 85 E122 (Hersteller BASF). Insbesondere können die textilweichmachenden Formulierungen zwischen 0,001 und 0,1 Gew.-% besonders bevorzugt 0,002 bis 0,05 Gew.-% eines oder mehrerer Farbstoffe oder Farbstoffmischungen enthalten, wobei sich die Gew.-% auf die gesamte Formulierung beziehen.
Als Viskositätsregulator zur Verringerung der Viskosität kann die textilweichmachende Formulierung ein Alkalimetall- oder Erdalkalimetallsalz, vorzugsweise Calciumchlorid, in einer Menge von 0,05 - 2 Gew.-% enthalten, wobei sich die Gew.-% auf die gesamte Formulierung beziehen. Als Viskositätsregler zur Erhöhung der Viskosität kann die textilweichmachende Formulierung einen aus dem Stand der Technik als geeignet bekannten Verdicker enthalten, wobei die aus WO 2007/125005 bekannten Polyurethanverdicker bevorzugt sind. Beispiele für geeignete Verdicker sind TEGO® Visco Plus 3030 (Hersteller Evonik Tego Chemie), Acusol® 880 und 882 (Hersteller Rohm & Haas), Rheovis® CDE (Hersteller BASF), Rohagit® KF 720 F (Hersteller Evonik Röhm GmbH) und Polygel® K100 von Neochem GmbH.
Als Entschäumer können alle für textilweichmachende Formulierungen aus dem Stand der Technik als geeignet bekannten Entschäumer eingesetzt werden. Beispiele für geeignete handelsübliche Entschäumer sind Dow Corning® DB-110A und TEGO® Antifoam® 7001 XP. Insbesondere können die textilweichmachenden Formulierungen zwischen 0,0001 und 0,05 besonders bevorzugt 0,001 bis 0,01 Gew.-% eines oder mehrerer unterschiedlicher Entschäumer enthalten, wobei sich die Gew.-% auf die gesamte Formulierung beziehen.
Als Konservierungsmittel kann die textilweichmachende Formulierung aus dem Stand der Technik als geeignet bekannte bakterizide und/oder fungizide Wirkstoffe enthalten, wobei wasserlösliche Wirkstoffe bevorzugt sind. Beispiele für geeignete handelsübliche Bakterizide sind Methylparaben, 2-Brom-2-nitro-1,3-propandiol, 2-Methyl-4-isothiazolin-3-on und 5-Chlor-2-methyl4-isothiazolin-3-on. Ebenso kann die textilweichmachende Formulierung als Konservierungsmittel einen Oxidationsinhibitor enthalten. Beispiele für geeignete handelsübliche Oxidationsinhibitoren sind Ascorbinsäure, 2,6-Di-tert-butyl-4-methlyphenol (BHT), Butylhydroxyanisol (BHA), Tocopherol und Propylgallat. Insbesondere können die textilweichmachenden Zusammensetzungen zwischen 0,0001 und 0,5, besonders bevorzugt 0,001 bis 0,2 Gew.-% eines oder mehrerer unterschiedlicher Konservierungsmittel enthalten. Insbesondere kann die textilweichmachende Formulierung zwischen 0,001 und 0,1, besonders bevorzugt 0,001 bis 0,01 Gew.-% eines oder mehrerer unterschiedlicher Oxidationsinhibitoren enthalten, wobei sich die Gew.-% auf die gesamte Formulierung beziehen.
Als organische Lösemittel kann die textilweichmachende Formulierung kurzkettige Alkohole, Glykole und Glykolmonoether enthalten, wobei Ethanol, 2-Propanol, 1,2-Propandiol und Dipropylenglycol bevorzugt wird. Insbesondere können die textilweichmachenden Zusammensetzungen zwischen 0,1 und 10, besonders bevorzugt 0,2 bis 5 Gew.-% eines oder mehrerer unterschiedlicher organischer Lösemittel enthalten, wobei sich die Gew.-% auf die gesamte Formulierung beziehen. Die textilweichmachende Formulierung kann eines oder mehrere nicht siloxanhaltige Polymere enthalten. Beispiele dafür sind Carboxymethylcellulose, Polyethylenglycol, Poylvinylalkohol, Poly(meth)acrylate), Polyethylenimine oder Polysaccharide. Insbesondere können die textilweichmachenden Formulierungen zwischen 0,01 und 25, besonders bevorzugt 0,1 bis 10 Gew.-% eines oder mehrerer unterschiedlicher nicht-siloxanhaltiger Polymere enthalten, wobei sich die Gew.-% auf die gesamte Formulierung beziehen.
Die textilweichmachende Formulierung kann darüber hinaus weitere, hier nicht aufgelistete, Zusatzstoffe enthalten, die für den Fachmann offensichtlich bzw. Stand der Technik sind.

Weitere Gegenstand der Erfindung ist die Verwendung der erfindungsgemäßen Mikroemulsionen in textilpflegenden Wasch- oder Reinigungsmitteln. Durch das Einbringen in ein Wasch- oder Reinigungsmittel steht dem Verbraucher ein textilpflegendes Wasch- oder Reinigungsmittel ("2in1"- Wasch- oder Reinigungsmittel) zur Verfügung und er braucht nicht zwei Mittel (Wasch- oder Reinigungsmittel und Weichspüler) zu dosieren, sowie keinen separaten Spülgang. Zusätzlich zu der textilpflegenden Formulierung und den Tensiden können die textilpflegenden Wasch- oder Reinigungsmittel weitere Inhaltsstoffe enthalten, die die anwendungstechnischen und/oder ästhetischen Eigenschaften des Wasch- oder Reinigungsmittels weiter verbessern. Bevorzugte Wasch- oder Reinigungsmittel enthalten zusätzlich einen oder mehrere Stoffe aus der Gruppe der Tenside, Gerüststoffe, Bleichmittel, Bleichaktivatoren, Enzyme, Parfüme, Parfümträger, Fluoreszenzmittel, Farbstoffe, Schauminhibitoren, Silikonöle, Antiredepositionsmittel, optische Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, neutrale Füllsalze sowie UV-Absorber. Insbesondere können die erfindungsgemäßen textilpflegenden Wasch- oder Reinigungsmitteln zwischen 0,001 und 90, besonders bevorzugt 0,01 bis 45 Gew.-% eines oder mehrerer der hier genannten weiteren Inhaltsstoffe enthalten, wobei sich die Gew.-% auf die gesamte Formulierung beziehen. Beispiele für einsetzbare Tenside werden in der WO 2007/115872, Seite 17, Zeile 28 bis Seite 21, Zeile 24 beschreiben. Beispiele für Gerüststoffe, Builder, Bleichmittel, Bleichaktivatoren, Bleichkatalysatoren und Enzyme werden in der WO 2007/115872, Seite 22, Zeile 7 bis Seite 25, Zeile 26 beschreiben. Antiredepositionsmittel, optische Aufheller, Vergrauungsinhibitoren, Farbübertragungsinhibitoren werden beispielhaft in der WO 2007/115872 auf Seite 26, Zeile 15 bis Seite 28, Zeile 2 beschrieben. Beispiele von Knitterschutzmitteln, antimikrobiellen Wirkstoffen, Germiziden, Fungiziden, Antioxidantien, Konservierungsmitteln, Antistatika, Bügelhilfsmitteln, UV-Absorbern werden in der WO 2007/115872 auf den Seiten 28, Zeile 14 bis Seite 30, Zeile 22.

Eine erfindungemäß bevorzugte Formulierung enthält die erfindungsgemäße Mikroemulsion in einer Menge von 0,1 Gew.-% bis 99 Gew.-%, bevorzugt in einer Menge von 0,5 Gew.-% bis 20 Gew.-%, besonders bevorzugt in einer Menge von 1,0 Gew.-% bis 10 Gew.-%, wobei sich die Gew.-% auf die Gesamtformulierung beziehen. Die erfindungsgemäße Formulierung, insbesondere die kosmetisch Formulierung, kann zum Beispiel mindestens eine zusätzliche Komponente enthalten, ausgewählt aus der Gruppe der
Emollients,
Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der EP2273966A1 entnommen werden. Diese Patentanmeldung wird hiermit als Referenz eingeführt und gilt somit als Teil der Offenbarung.
Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Weitere Gegenstand der Erfindung ist die Verwendung der erfindungsgemäßen Mikroemulsionen als Additiv für Lacke und Farben, insbesondere für wasserbasierte Lacke und Farben, bevorzugt solche, in denen Polyurethandispersionen als Bindemittel eingesetzt werden. Durch die Zugabe der Mikroemulsion erhält der Anwender Lacke und Farben, die sich nach Auftragung durch geringere (Haft-)Gleitreibung und veränderten Griff beziehungsweise Haptik auszeichnen.
Lacke und Farben, insbesondere zur Beschichtung von Textil, Metall, Leder, Kunststoff, Papier, Pappe und Holz, enthaltend mindestens eine erfindungsgemäße Mikroemulsion oder mindestens eine Mikroemulsion erhältlich nach dem erfindungsgemäßen Verfahren, wobei diese Lacke und Farben bevorzugt als Bindemittel eine wässrige Polyurethandispersion enthalten, sind ebenfalls Gegenstand der vorliegenden Erfindung
Zusätzlich zu dem Bindemittel und der erfindungsgemäßen Mikroemulsion kann der Lack oder die Farbe weitere Inhaltsstoffe enthalten, die die anwendungstechnischen und/oder ästhetischen Eigenschaften der Beschichtung weiter verbessern. Bevorzugte Lacksysteme enthalten zusätzlich einen oder mehrere Stoffe aus der Gruppe der Primär- oder Co-Bindemittel, Vernetzer, Härter,Tenside, Substratnetzmittel, Dispergieradditive, Rheologieadditive, Entschäumer, Entlüfter, sowie anorganische oder aber auch organische Pigmente, Farbstoffe, Gleit- und Verlaufsadditive, Füllstoffe, Mattierungsmittel, Perlpolymerisate, natürliche und synthetische Wachse, griffverbesserende Mittel, antimikrobielle Wirkstoffe, Germizide, Fungizide, Antioxidantien, Konservierungsmittel, UV-Stabilisatoren und polare Lösemittel. Erfindungemäß bevorzugte Lacke oder Farbeen enthalten die erfindungsgemäße Mikroemulsion in einer Menge von 0,1 Gew.-% bis 99 Gew.-%, bevorzugt in einer Menge von 0,5 Gew.-% bis 20 Gew.-%, besonders bevorzugt in einer Menge von 1,0 Gew.-% bis 10 Gew.-%, wobei sich die Gew.-% auf die Gesamtformulierung beziehen.

Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

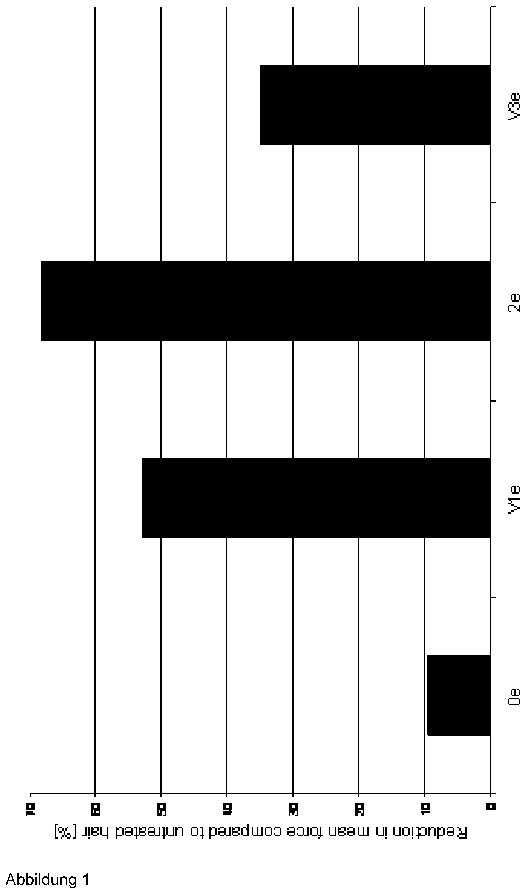

Abbildung 1 ist Bestandteil der Beispiele und zeigt die Erniedrigung der Reibung durch Anwendung von Konditioniermitteln.

### Beispiele

### Siloxan 1: Polysiloxan T-Quat mit N=121:

Entsprechend Beispiel 4 aus EP 1887024 wurde ein quaternäres Polysiloxan hergestellt, jedoch mit einer Gesamtsiloxankettenlänge von N=121. Es wurde dabei ein hochviskoses, leicht gelbliches Produkt mit folgender statistischen Struktur erhalten.

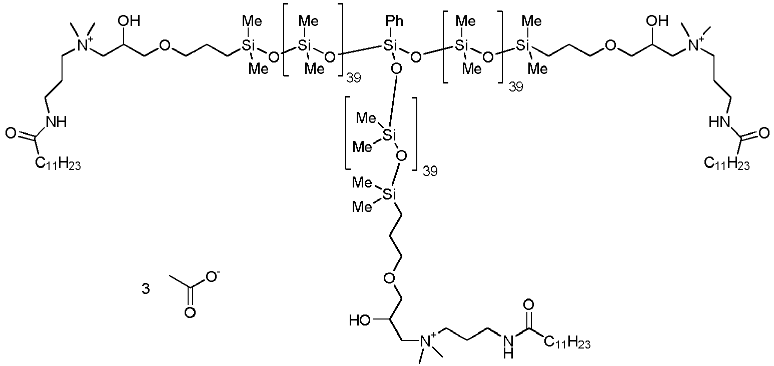

### Siloxan 2: Polysiloxan T-Quat mit N=151:

Entsprechend Beispiel 4 aus EP 1887024 wurde ein quaternäres Polysiloxan hergestellt, jedoch mit einer Gesamtsiloxankettenlänge von N=151 und mit Milchsäure anstelle von Essigsäure. Es wurde dabei ein hochviskoses, leicht gelbliches Produkt mit folgender statistischen Struktur erhalten.

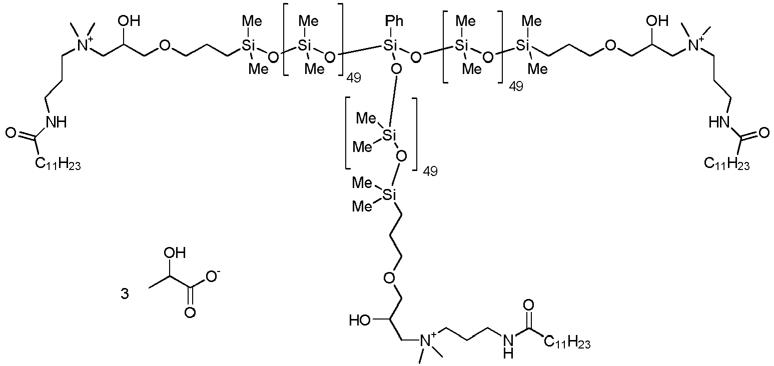

### Siloxan 3: Polysiloxan T-Quat mit N=211:

Entsprechend Beispiel 4 aus EP 1887024 wurde ein quaternäres Polysiloxan hergestellt, jedoch mit einer Gesamtsiloxankettenlänge von N=211. Es wurde dabei ein hochviskoses, leicht gelbliches Produkt mit folgender statistischen Struktur erhalten.

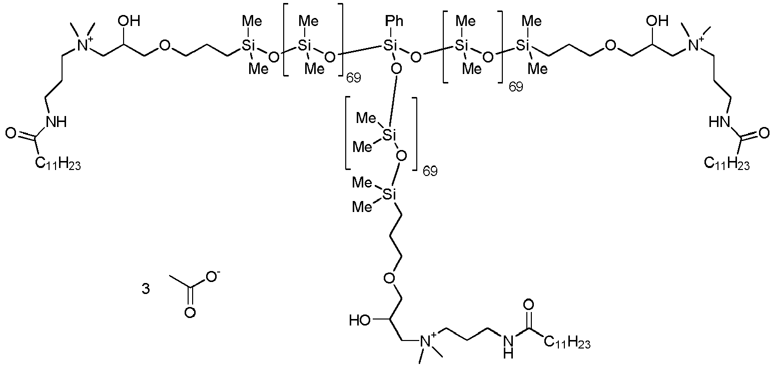

### Siloxan 4: Polysiloxan Multi-T-Quat mit N=250:

Entsprechend Beispiel 2 aus PCT/EP2010/070071 wurde ein quaternäres Polysiloxan mit 5 T-Einheiten hergestellt, jedoch mit einer Gesamtsiloxankettenlänge von N=250. Es wurde dabei ein sehr viskoses, gelbliches Produkt mit folgender statistischen Formel erhalten. (R₂Me₂SiO_{1/2})₇ (Me₂SiO_{1/2})₂₃₈ (MeSiO_{3/2})₅ mit R² =

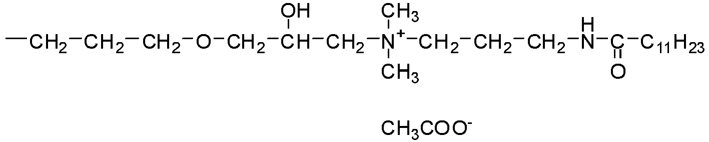

### Siloxan 5: Polysiloxan Multi-T-Quat mit N=350:

Entsprechend Beispiel 2 aus PCT/EP2010/070071 wurde ein quaternäres Polysiloxan mit 5 T-Einheiten hergestellt, jedoch mit einer Gesamtsiloxankettenlänge von N=350. Es wurde dabei ein klares, sehr viskoses, gelbliches Produkt mit folgender statistischen Formel erhalten. (R₂Me₂SiO_{1/2})₇ (Me₂SiO_{1/2})₃₃₈ (MeSiO_{3/2})₅ mit R₂ =

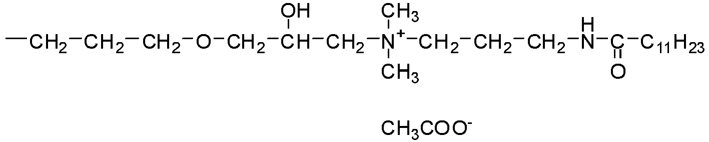

### Siloxan 6: Polysiloxan T-Quat mit N=61:

Entsprechend Beispiel 4 aus EP 1887024 wurde ein quaternäres Polysiloxan hergestellt, jedoch mit einer Gesamtsiloxankettenlänge von N=61. Es wurde dabei ein hochviskoses, leicht gelbliches Produkt mit folgender statistischen Struktur erhalten.

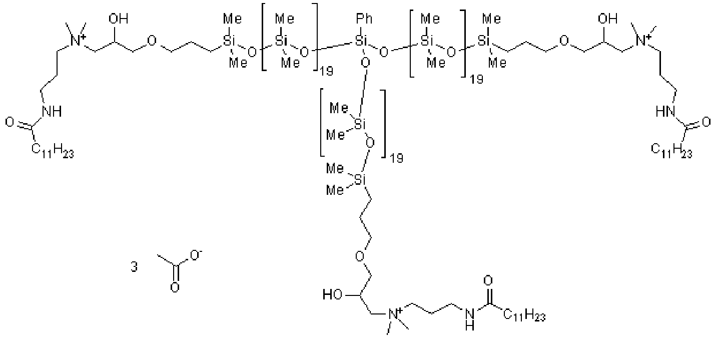

### Formulierungsbeispiele Mikroemulsionen

| **Bestandteil / Gew.-%** | **ME1** | **ME2** | **ME3** |
|---|---|---|---|
| Siloxan 2 | 25,02 | 21,20 | 27,90 |
| VARISOFT® 300, Evonik Goldschmidt GmbH (Cetrimonium Chloride) | 9,18 | 10,23 | 5,68 |
| ANTIL® Soft SC, Evonik Goldschmidt GmbH (Sorbitan Sesquicaprylate) | 7,63 | | |
| Imbentin® U/050, Kolb (Undeceth-5) | | 9,96 | |
| Dermosoft® GMCY, Dr. Straetmans (Glyceryl Caprylate) | | | 11,25 |
| Isopropanol | 6,23 | 5,30 | |
| DOWANOL® TPM, Dow Chemical (Tripropylenglykolmonomethylether) | | | 6,97 |
| Wasser | 51,94 | 53,31 | 48,20 |

| **Bestandteil / Gew.-%** | **ME4** | **ME5** | **ME6** | **ME7** |
|---|---|---|---|---|
| Siloxan 2 | 25,70 | 31,00 | 30,00 | 30,00 |
| Dipropylenglykol | 8,10 | 2,10 | 5,00 | 10,00 |
| Dermosoft® GMCY, Dr. Straetmans (Glyceryl Caprylate) | 7,70 | 9,50 | 9,00 | 9,10 |
| Glycerin | 10,40 | | | |
| VARISOFT® 300, Evonik Goldschmidt GmbH (Cetrimonium Chlorid) | 2,50 | | 3,50 | |
| VARISOFT® PATC, Evonik Goldschmidt GmbH (Palmitamidopropyltrimonium Chlorid) | | 3,20 | | 3,50 |
| Phenoxyethanol | 0,80 | 0,90 | 0,80 | 0,80 |
| Wasser | 44,10 | 53,00 | 51,40 | 46,40 |
| Caprinsäure | 0,40 | | | |
| Zitronensäure 40%ig | 0,30 | | | |
| NaCl | | 0,30 | 0,30 | 0,20 |

| **Bestandteil / Gew.-%** | **ME8** | **ME9** |
|---|---|---|
| Siloxan 2 | 30,00 | 30,00 |
| 1,2-Hexandiol | 4,80 | |
| Hexylenglykol | | 8,20 |
| Glyceryl Caprylat | 6,30 | 9,20 |
| VARISOFT® PATC, Evonik Goldschmidt GmbH (Palmitamidopropyltrimonium Chlorid) | 5,00 | 3,60 |
| Phenoxyethanol | 0,80 | 0,80 |
| Wasser | 53,10 | 48,20 |

| **Bestandteil / Gew.-%** | **ME10** | **ME11** |
|---|---|---|
| Siloxan 2 | 25,20 | 26,00 |
| Dipropylenglykol | | 10,30 |
| Hexylenglykol | 8,00 | |
| Dermosoft® GMCY, Dr. Straetmans (Glyceryl Caprylate) | 9,20 | |
| Glyceryl Caprylat | | 6,20 |
| TEGO® Betain F50, Evonik Goldschmidt GmbH (Cocoamidopropylbetain) | | 5,20 |
| VARISOFT® PATC, Evonik Goldschmidt GmbH (Palmitamidopropyltrimonium Chlorid) | 3,60 | 3,90 |
| Phenoxyethanol | 0,80 | 0,80 |
| Wasser | 53,20 | 47,60 |

| **Bestandteil / Gew.-%** | **ME12** | **ME13** | **ME14** |
|---|---|---|---|
| Siloxan 2 | 30,36 | 28,20 | 25,57 |
| Propylenglykol | 10,12 | | |
| Hexylenglykol | | 9,40 | |
| Butylenglykol | | | 8,52 |
| Glyceryl Caprylat | 6,07 | 5,60 | 5,30 |
| TEGO® Betain F50, Evonik Goldschmidt GmbH (Cocoamidopropylbetain) | 10,12 | 9,40 | 8,90 |
| VARISOFT® PATC, Evonik Goldschmidt GmbH (Palmitamidopropyltrimonium Chlorid) | 4,05 | 3,80 | 3,60 |
| Phenoxyethanol | 0,81 | 0,80 | 0,76 |
| Wasser | 38,47 | 42,80 | 47,35 |

| **Bestandteil / Gew.-%** | **ME15** | **ME16** | **ME17** |
|---|---|---|---|
| Siloxan 1 | 25,62 | 30,00 | |
| Siloxan 6 | | | 30,03 |
| Dipropylenglykol | | 10,00 | 5,01 |
| Dermosoft® GMCY, Dr. Straetmans (Glyceryl Caprylate) | 9,67 | 9,00 | 9,01 |
| Glycerin | 13,84 | | |
| Caprinsäure | 0,64 | | |
| VARISOFT® PATC, Evonik Goldschmidt GmbH (Palmitamidopropyltrimonium Chlorid) | 3,33 | 3,50 | 3,50 |
| Phenoxyethanol | 0,83 | 0,80 | 0,80 |
| Wasser | 46,07 | 46,47 | 51,39 |
| NaCl | | 0,23 | 0,26 |

| **Bestandteil / Gew.-%** | **ME18** | **ME19** | **ME20** | **ME21** |
|---|---|---|---|---|
| Siloxan 2 | 30,00 | 40,12 | 46,30 | |
| Siloxan 5 | | | | 30,00 |
| Dipropylenglykol | 9,00 | 7,08 | 14,81 | |
| Propylenglykol | | | | 10,00 |
| TEGOSOFT® PC 31, Evonik Goldschmidt GmbH (Polyglyceryl-3 Caprate) | 11,00 | 10,00 | 12,22 | 10,00 |
| TEGO® Betain F 50, Evonik Goldschmidt GmbH (Cocoamdopropyl Betaine) | 10,00 | 10,00 | 4,63 | 5,00 |
| VARISOFT® PATC, Evonik Goldschmidt GmbH (Palmitamidopropyltrimonium Chloride) | 3,60 | 3,60 | 1,48 | 2,40 |
| Wasser | 36,40 | 29,20 | 20,56 | 42,6 |

| **Bestandteil / Gew.-%** | **ME22** | **ME23** | **ME24** | **ME25** |
|---|---|---|---|---|
| Siloxan 2 | 26,19 | 24,11 | 30,31 | 33,04 |
| Dipropylenglykol | 13,15 | 11,87 | | |
| 1,2-Hexandiol | | | 14,43 | 15,22 |
| Dermosoft® GMCY, Dr. Straetmans (Glyceryl Caprylate) | 10,66 | | | |
| TEGOSOFT® CT, Evonik Goldschmidt GmbH (Caprylic/Capric Triglyceride) | | | 3,00 | |
| TEGOSOFT® M, Evonik Goldschmidt GmbH (Isopropyl Myristate) | | 7,45 | | 1,22 |
| VARISOFT® 432 PPG, Evonik Goldschmidt GmbH (Dicetyldimoniumchloride) | 3,71 | 16,23 | 5,77 | 3,52 |
| Wasser | 46,29 | 40,34 | 46,49 | 47,00 |

| **Bestandteil / Gew.-%** | **ME26** | **ME27** | **ME28** | **ME29** |
|---|---|---|---|---|
| Siloxan 2 | 28,85 | 27,08 | 35,62 | 26,87 |
| Dipropylenglykol | 5,77 | 13,02 | | 13,33 |
| Butyldiglykol | | | 10,27 | |
| Dermosoft® GMCY, Dr. Straetmans (Glyceryl Caprylate) | | | | 10,66 |
| ANTIL® Soft SC, Evonik Goldschmidt GmbH (Sorbitan Sesquicaprylate) | | 10,42 | | |
| TEGO® Cosmo P 813, Evonik Goldschmidt GmbH (Polyglyceryl-3-Caprylate) | 9,62 | | | |
| CAPRYOL 90™, Gattefossé (Propylene Glycol Monocaprylate) | | | 10,27 | |
| TEGO® Betain F 50, Evonik Goldschmidt GmbH (Cocoamdopropyl Betaine) | 5,77 | | | |
| VARISOFT® 432 CG, Evonik Goldschmidt GmbH (Dicetyldimonium Chloride) | | | | 3,72 |
| VARISOFT® PATC, Evonik Goldschmidt GmbH (Palmitamidopropyltrimonium Chlorid) | 2,30 | 5,21 | 6,85 | |
| Wasser | 47,69 | 44,27 | 36,99 | 45,42 |

| **Bestandteil / Gew.-%** | **ME30** | **ME31** | **ME32** | **ME33** | **ME34** |
|---|---|---|---|---|---|
| Siloxan 2 | 24,11 | | | | |
| Siloxan 1 | | | | | 38,46 |
| Siloxan 3 | | | 14,80 | | |
| Siloxan 4 | | 30,00 | | | |
| Siloxan 5 | | | | 20,50 | |
| TEGO® Alkanol TD6, Evonik Goldschmidt GmbH (POE-(6)-isotridecyl Alcohol) | 8,74 | 12,00 | 12,00 | 11,80 | 11,54 |
| Wasser | 58,65 | 49,60 | 64,80 | 59,30 | 28,85 |
| Isopropanol | 8,50 | 8,40 | 8,40 | 8,40 | 21,15 |

| **Bestandteil / Gew.-%** | **ME35** | **ME36** |
|---|---|---|
| Siloxan 2 | 21,1 | |
| Siloxan 5 | | 20,0 |
| TEGO® Alkanol TD6, Evonik Goldschmidt GmbH (POE-(6)-isotridecyl Alcohol) | 11,6 | 12,00 |
| Lutensol TO12, BASF AG (POE-(12)-isotridecyl Alcohol) | 3,8 | 3,8 |
| Wasser | 58,2 | 56,2 |
| Dipropylenglykol | 5,3 | 8,0 |

### Anwendungstechnik Kosmetik:

Im Folgenden wurden 4 verschiedene Produkte in kosmetischen Formulierungen ausgetestet:
Die erfindungsgemäße Mikroemulsion ME18 ist wie folgt zusammengesetzt (s.o.):

| **Bestandteil / Gew.-%** | **ME18** |
|---|---|
| Siloxan 2 | 30,00 |
| Dipropylenglykol | 9,00 |
| TEGOSOFT® PC 31, Evonik Goldschmidt GmbH (Polyglyceryl-3 Caprate) | 11,00 |
| TEGO® Betain F 50, Evonik Goldschmidt GmbH (Cocoamdopropyl Betaine) | 10,00 |
| VARISOFT® PATC, Evonik Goldschmidt GmbH (Palmitamidopropyltrimonium Chloride) | 3,60 |
| Wasser | 36,40 |

Die Mikroemulsion hat einen Silikon-Aktivwirkstoffgehalt von 30%.

### Vergleichsbeispielprodukt 2 (nicht erfindungsgemäß):

Hierzu wurde Siloxan 2 in 15% Dipropylenglycol gelöst (d.h. Aktivgehalt von 85% Silikon-Aktivwirkstoff).

### Vergleichsbeispiel 3 (nicht erfindungsgemäß):

Aminogruppenhaltiges Siloxan DC 2-8566 (kommerziell erhältlich bei Dow Corning, INCI: Amodimethicone). Das Produkt besteht aus 100% Silikon-Aktivwirkstoff.

### Vergleichsbeispiel 4 (nicht erfindungsgemäß):

Mikroemulsion mit quaternären Siloxan DC 5-7113 (kommerziell erhältlich bei Dow Corning, INCI: Silicone Quaternium-16 (and) Undeceth-11 (and) Butyloctanol (and) Undeceth-5). Die Mikroemulsion hat einen Silikon-Aktivwirkstoffgehalt von 22%.

### Anwendungstechnische Eigenschaften

Die Formulierungsbestandteile sind in den Zusammensetzungen in Form der allgemein anerkannten INCI-Nomenklatur unter Verwendung der englischen Begriffe benannt. Alle Konzentrationen in den Anwendungsbeispielen sind in Gewichtsprozent angegeben.

### 1.) Austestung der Konditionierung von Haut (Hautpflegeleistung) und der Schaumeigenschaften mittels eines Handwaschtests:

Zur Bewertung der Konditionierung von Haut (Hautpflegeleistung) und der Schaumeigenschaften der erfindungsgemäßen Mikroemulsion ME18 in wässrigen, tensidischen Formulierungen wurden sensorische Handwaschtests im Vergleich zu den Vergleichsbeispielen 2 und 3 nach dem Stand der Technik durchgeführt.

Das Vergleichsbeispiel 3 ist in der Industrie als Pflegewirkstoff weit verbreitet und gilt als hochwirksamer Pflegewirkstoff in wässrigen, tensidischen Formulierungen.
Das Vergleichsbeispiel 2 enthält den gleichen Silikonwirkstoff wie die Mikroemulsion ME18.
Eine Gruppe bestehend aus 10 trainierten Prüfpersonen wusch sich dabei definiert die Hände und bewertete Schaumeigenschaften und Hautgefühl anhand einer Notenskala von 1 (schlecht) bis 5 (sehr gut).

Die eingesetzten Produkte wurden jeweils in einer standardisierten Tensidformulierung (Tabelle 1) getestet.
Als Kontrollformulierung 0b wird eine Formulierung ohne Zusatz eines organomodifizierten Siloxans verwendet.

**Tab. 1: Testformulierungen für Handwaschtest.**

| **Formulierungsbeispiele** | **0b** | **1b** | **V2b** | **V3b** |
|---|---|---|---|---|
| Texapon NSO®, 28%-ig, Cognis (INCI: Sodium Laureth Sulfate) | 32% | 32% | 32% | 32% |
| TEGO® Betain F 50, 38%-ig, Evonik Goldschmidt GmbH (INCI: Cocamidopropyl Betaine) | 8% | 8% | 8% | 8% |
| NaCl | 2% | 2% | 2% | 2% |
| Wasser, demineralisiert | ad 100,0% | | | |
| Mikroemulsion ME18 (erfindungsgemäß) | | 1,67% | | |
| Vergleichsbeispiel 2 (nicht erfindungsgemäß) | | | 0,59% | |
| Vergleichsbeispiel 3 (nicht erfindungsgemäß) | | | | 0,5% |

Eine Austestung mit Formulierungsbeispiel V2b konnte nicht erfolgen, da starke Separation auftrat. Der Silikonwirkstoff kann in die tensidische Formulierung nicht stabil eingearbeitet werden.

Die sensorischen Testergebnisse sind in Tabelle 2 zusammengefasst.

**Tab. 2: Ergebnisse des Handwaschtests**

| **Testformulierung** | **0b** | **1b** | **V3b** |
|---|---|---|---|
| Anschäumverhalten | 3,0 | 4,0 | 3,3 |
| Schaumvolumen | 2,8 | 3,5 | 2,9 |
| Schaumcremigkeit | 2,3 | 3,2 | 3,0 |
| Hautgefühl während des Waschens | 2,8 | 4,0 | 3,7 |
| Hautglätte | 1,4 | 3,5 | 2,9 |
| Hautweichheit | 2,0 | 3,3 | 2,9 |
| Hautglätte nach 3 min. | 2,6 | 3,9 | 3,6 |
| Hautweichheit nach 3 min. | 2,5 | 3,8 | 3,5 |

In Tabelle 2 sind die Ergebnisse des Handwaschtests dargestellt. Anhand der Messergebnisse wird ersichtlich, dass die erfindungsgemäße Formulierung 1b unter Verwendung der erfindungsgemäßen Mikroemulsion ME18 in allen Applikationseigenschaften im Vergleich zur Vergleichsformulierung V2b nach dem Stand der Technik überlegen ist.
Vor diesem Hintergrund sind die Ergebnisse der erfindungsgemäßen Formulierung 1b als sehr gut zu bezeichnen.
Anhand der Messwerte ist ersichtlich, dass die erfindungsgemäße Mikroemulsion ME18 in der Formulierung 1b zu einer Verbesserung der Hauteigenschaften und Schaumeigenschaften im Vergleich zum Vergleichsbeispiel 3 in der Formulierung V3b führt.
Ferner kann man den Messwerten entnehmen, dass die Kontrollformulierung 0b ohne eine Siliconverbindung schlechtere Messwerte als die Formulierungen 1b und V3b aufweist.
Ferner ist die Tatsache, dass Vergleichsbeispiel 2 nicht in die Vergleichsformulierung V2b eingearbeitet werden kann, obwohl Vergleichsbeispiel 2 den gleichen Silikonwirkstoff wie die erfindungsgemäße Mikroemulsion ME18 enthält, ein deutlicher Beweis dafür, dass die erfindungsgemäßen Mikroemulsionen eine deutliche Verbesserung des Stands der Technik darstellen, da die erfindungsgemäßen Mikroemulsionen die Verwendung von Silikonwirkstoffen ermöglichen, die nach dem Stand der Technik nicht in tensidischen Formulierungen verwendet werden konnten.

### 2.) Austestung der Konditionierung von Haar mittels Sensoriktests:

Für die anwendungstechnische Beurteilung der Konditionierung von Haar wurden die erfindungsgemäße Mikroemulsion ME18 und das Vergleichsbeispiel 3 in einfachen kosmetischen Formulierungen (Shampoo und Haarspülung) eingesetzt.

Die Anwendungseigenschaften beim Einsatz in einem Shampoo wurden in den folgenden Rezepturen überprüft:

**Tab. 3: Shampooformulierungen zur Austestung der haarkonditionierenden Eigenschaften.**

| **Formulierungsbeispiele** | **0c** | **1c** | **V2c** |
|---|---|---|---|
| Texapon NSO®, 28%-ig, Cognis (INCI: Sodium Laureth Sulfate) | 32% | 32% | 32% |
| TEGO® Betain F 50, 38%-ig, Evonik Goldschmidt GmbH (INCI: Cocamidopropyl Betaine) | 8% | 8% | 8% |
| Jaguar 162, Rhodia (INCI: Guar Hydroxypropyl trimonium Chloride) (Kationisches Polymer zur Verbesserung der Wirksamkeit von Konditioniermitteln) | 0,3% | 0,3% | 0,3% |
| Wasser, demineralisiert | ad 100,0% | | |
| Zitronensäure | ad. pH 6,0 ± 0,3 | | |
| Mikroemulsion ME18 (erfindungsgemäß) | | 1,67% | |
| Vergleichsbeispiel 3 (nicht erfindungsgemäß) | | | 0,5% |

Zur Bewertung der Eigenschaften der Shampooformulierung wurde im Testablauf keine Nachbehandlung mit einer Spülung durchgeführt.

Die Anwendungseigenschaften beim Einsatz in Haarspülungen wurden in den folgenden Rezepturen überprüft:

**Tab. 4: Haarspülungformulierungen zur Austestung der haarkonditionierenden Eigenschaften.**

| **Formulierungsbeispiele** | **0d** | **1d** | **V2d** |
|---|---|---|---|
| TEGINACID® C, Evonik Goldschmidt GmbH (INCI: Ceteareth-25) | 0,5% | 0,5% | 0,5% |
| TEGO®Alkanol 16, Evonik Goldschmidt GmbH (INCI: Cetyl Alcohol) | 4% | 4% | 4% |
| VARISOFT® 300, 30%-ig, Evonik Goldschmidt GmbH (INCI: Cetrimonium Chloride) | 3,3% | 3,3% | 3,3% |
| Wasser, demineralisiert | ad. 100,0% | | |
| Zitronensäure | ad. pH 4,0 ± 0,3 | | |
| Mikroemulsion ME18 (erfindungsgemäß) | | 1,67% | |
| Vergleichsbeispiel 3 (nicht erfindungsgemäß) | | | 0,5% |

Die Vorbehandlung der Haare erfolgt im Falle der Eigenschaftsprüfung von Haarspülungen durch ein Shampoo, welches keine Konditioniermittel enthält.

Für die anwendungstechnische Beurteilung werden Haartressen, die für sensorische Tests verwendet werden, durch eine Dauerwellbehandlung und eine Bleichbehandlung standardisiert vorgeschädigt. Dazu werden friseurübliche Produkte verwendet. Der Testablauf, die verwendeten Basismaterialien sowie die Details der Beurteilungskriterien sind in DE 103 27 871 beschrieben.

Standardisierte Behandlung von vorgeschädigten Haartressen mit konditionierenden Proben:
Die, wie oben beschrieben, vorgeschädigten Haartressen werden wie folgt mit dem oben beschriebenen Shampoo oder der oben beschriebenen konditionierenden Spülung behandelt:
Die Haartressen werden unter fließendem, warmem Wasser benetzt. Das überschüssige Wasser wird leicht von Hand ausgedrückt, dann wird das Shampoo aufgebracht und sanft im Haar eingearbeitet (1 ml/Haartresse (2 g)). Nach einer Verweilzeit von 1 min wird das Haar für 1 min gespült.
Gegebenenfalls wird direkt im Anschluss die Spülung aufgebracht und sanft im Haar eingearbeitet (1 ml/Haartresse (2 g)). Nach einer Verweilzeit von 1 min wird das Haar für 1 min gespült.

Vor der sensorischen Beurteilung wird das Haar an der Luft bei 50% Luftfeuchtigkeit und 25 °C für mindestens 12 h getrocknet.

### Beurteilungskriterien:

Die sensorischen Bewertungen erfolgen nach Noten, die auf einer Skala von 1 bis 5 vergeben werden, wobei 1 die schlechteste und 5 die beste Bewertung ist. Die einzelnen Testkriterien erhalten jeweils eine eigene Bewertung.
Die Testkriterien sind: Nasskämmbarkeit, Nassgriff, Trockenkämmbarkeit, Trockengriff, Aussehen/Glanz.

In der folgenden Tabelle werden die Ergebnisse der sensorischen Beurteilung der wie oben beschrieben durchgeführten Behandlung der Haarstränchen mit der erfindungsgemäßen Formulierung 1c, der Vergleichsformulierung V2c und der Kontrollformulierung 0c (Placebo ohne Testsubstanz) verglichen.

**Tab. 5: Ergebnisse der Konditionierung von Haar aus Shampooformulierung**

| | Nasskämmbarkeit | Nassgriff | Trockenkämmbarkeit | Trockengriff | Glanz |
|---|---|---|---|---|---|
| Erfindungsgemäße Formulierung 1c | 3,7 | 3,7 | 3,5 | 4,3 | 4,2 |
| Vergleichsformulierung V2c (nicht erfindungsgemäß) | 3,2 | 3,1 | 3,1 | 3,8 | 3,3 |
| Kontrollformulierung 0c (Placebo) | 2,4 | 2,4 | 2,6 | 3,2 | 2,5 |

Die Ergebnisse zeigen in überraschender Weise, dass die erfindungsgemäße Formulierung 1c mit der erfindungsgemäßen Mikroemulsion ME18 signifikant bessere Bewertungen erhält als die Vergleichsformulierung V2c mit dem Vergleichsbeispiel 3 nach Stand der Technik. Besonders deutlich ist die gute Bewertung der Glanzeigenschaften aller erfindungsgemäßen Formulierungen hervorzuheben.

**Tab. 6: Ergebnisse der Konditionierung von Haar aus Haarspülungformulierungen**

| | Nasskämmbarkeit | Nassgriff | Trockenkämmbarkeit | Trockengriff | Glanz |
|---|---|---|---|---|---|
| Erfindungsgemäße Formulierung 1d | 4,9 | 5,0 | 4,8 | 4,8 | 4,4 |
| Vergleichsformulierung V2d (nicht erfindungsgemäß) | 4,3 | 4,4 | 4,5 | 4,5 | 3,9 |
| Kontrollformulierung 0d | 3,8 | 3,9 | 3,9 | 3,8 | 2,9 |

Auch in der Anwendung Haarspülung zeigt die erfindungsgemäße Formulierung 1d mit der erfindungsgemäßen Mikroemulsion ME18 in der sensorischen Beurteilung sehr gute kosmetische Bewertungen. Dabei wurden die bereits sehr guten Eigenschaften der Vergleichsformulierung V2d mit dem Vergleichsbeispiel 3 durch die erfindungsgemäße Formulierung 1d mit der erfindungsgemäßen Mikroemulsion noch weiter gesteigert.
Eine signifikant bessere Bewertung wird auch beim Glanz durch die Verwendung der erfindungsgemäßen Formulierung 1d erreicht.

### 3.) Austestung der Reibungswerte auf trockenem Haar mittels Friction Test:

Die Konditionierwirkung der Produkte auf trockenem Haar wurde mit Hilfe einer Reibungskraft-Messmethode untersucht (siehe auch US 2009/0324530). Hierzu wurde ein Instrument der Firma Instron (Instron 5942, Instron Deutschland GmbH, Pfungstadt, Deutschland) verwendet.
Das Instrument misst die Kraft, die nötig ist, ein Schlitten über eine Echthaartresse zu ziehen. Die Differenz der Kraft aus der Messung vor und der Messung nach der Behandlung mit dem Konditionieragenz ergibt die Reibwerterniedrigung, und damit einen objektiv erfassten Wert für die Qualität des verwendeten Konditionierers.
Der Schlitten von 200 g Gewicht und mit den Maßen 6 x 7cm x 0.5 cm wurde dabei mit einer Vollgummi-Oberfläche ausgestattet. Für jede Haartresse wird diese Oberfläche erneuert. Es wurden durch Bleichung vorgeschädigte und vorgewaschene Echthaartressen (7 cm breit, 18 cm freie Haarlänge, ca. 8.5 g) verwendet.

### Behandlung der Haartressen:

Die Produkte wurden aus einer Haarspülung angewendet. Die erfindungsgemäße Mikroemulsion wurde im Vergleich zu den Vergleichsbeispielen 2 und 4 untersucht. Die entsprechenden Formulierungen sind in Tabelle 7 zusammengefasst.

**Tab. 7: Haarspülung Formulierungen zur Austestung der Reibung auf Haaren.**

| **Formulierungsbeispiele** | **0e** | **V1e** | **2e** | **V3e** |
|---|---|---|---|---|
| TEGO® Alkanol 16, Evonik Goldschmidt GmbH (INCI: Cetyl Alcohol)) | 5,00% | 5,00% | 5,00% | 5,00% |
| TEGINACID®C, Evonik Goldschmidt GmbH (INCI: Ceteareth-25) | 0,50% | 0,50% | 0,50% | 0,50% |
| VARISOFT® 300, 30%-ig, Evonik Goldschmidt GmbH (INCI: Cetrimonium Chloride) | 3,30% | 3,30% | 3,30% | 3,30% |
| Vergleichsbeispiel 4 | | 2,27% | | |
| Mikroemulsion ME18 (erfindungsgemäß) | | | 1,67% | |
| Vergleichsbeispiel 2 | | | | 0,59% |
| Wasser | 90,75% | 88,45% | 89,05% | 90,15% |
| Neolone PE | 0,45% | 0,45% | 0,45% | 0,45% |

Die Haarspülungsformulierungen wurden in einer Konzentration von 0,5 g / 2 g Haar auf die Haartresse aufgetragen, gleichmäßig innerhalb 1 min verteilt und eingepflegt, für 1 min einwirken gelassen und mit 38 °C warmen Wasser für 3 min ausgespült. Die Haartressen wurden über Nacht bei 22 °C und 50% rel. Luftfeuchtigkeit trocknen gelassen, bevor sie mittels der oben beschriebenen Methode am Instron-Kraftmessgerät vermessen wurden.
Die erhaltenen Reibungswert-Erniedrigungen durch die Anwendung der Konditionierer sind in Abbildung 1 dargestellt.

Anhand der Messwerte wird ersichtlich, dass eine deutliche Reduktion der Reibung mit den Vergleichformulierungen V1e und V3e mit den Vergleichsbeispielen 4 und 2 und der erfindungsgemäßen Formulierung 2e mit der erfindungsgemäßen Mikroemulsion 1 erzielt werden können.
Es wird ferner ersichtlich, dass eine ausgeprägtere Reduktion der Reibung mit der erfindungsgemäßen Formulierung 2e mit der erfindungsgemäßen Mikroemulsion ME18 als mit der Vergleichsformulierung V1e mit dem Vergleichsbeispiel 4 nach dem Stand der Technik erzielt werden kann. Folglich ist das nicht erfindungsgemäße Vergleichsbeispiel 4 (Mikroemulsion nach dem Stand der Technik) weniger effektiv als die erfindungsgemäße Formulierung 2e mit der erfindungsgemäßen Mikroemulsion ME18.

Die Formulierung V 3e mit dem Vergleichsbeispiel 2 enthält die gleiche quaternäre Siloxanstruktur wie die erfindungsgemäße Formulierung 2e mit der erfindungsgemäßen Mikroemulsion ME18. Überraschenderweise ist mit der Formulierung 2e eine deutlich ausgeprägtere Reduktion der Reibung gefunden worden. Folglich stellt die Mikroemulsion ME18 eine deutliche Verbesserung des Standes der Technik dar.

### Weitere Formulierungsbeispiele:

Die in den nachfolgenden Tabellen angegebenen Formulierungsbeispiele zeigen exemplarische Vertreter einer Vielzahl von möglichen erfindungsgemäßen Zusammensetzungen.

Falls die Herstellung der Formulierung zuvor die getrennte Zubereitung bzw. Mischung von Formulierungsbestandteilen erfordert, wird dieses als mehrphasige Zubereitung bezeichnet.

Falls eine zweiphasige Herstellung erforderlich ist, werden die beiden Phasen mit A und B in den angegebenen Tabellen gekennzeichnet. Bei dreiphasigen Prozessen werden die drei Phasen mit A, B und C benannt. Wenn nicht anders angegeben handelt es sich bei den Angaben in den folgenden Tabellen um Angaben in Gew.-%.

**Formulierungsbeispiel 1) Clear Shampoo**

| | |
|---|---|
| TEXAPON® NSO, Cognis, 28%-ig (INCI: Sodium Laureth Sulfate) | 32,00% |
| Mikroemulsion ME7 | 2,50% |
| Perfume | 0,50% |
| Water | 55,50% |
| TEGO® Betain F 50, Evonik Goldschmidt GmbH, 38%-ig (INCI: Cocamidopropyl Betaine) | 8,00% |
| ANTIL® 171 Evonik Goldschmidt GmbH (INCI: PEG-18 Glyceryl Oleate/Cocoate) | 1,00% |
| NaCl | 0,50% |
| Preservative | q.s. |

**Formulierungsbeispiel 2) Shampoo, PEG- & sulfate free**

| | |
|---|---|
| REWOTERIC® AM C, Evonik Goldschmidt GmbH, 32%-ig, (INCI: Sodium Cocoamphoacetate) | 15,00% |
| Plantapon ACG 50, Cognis (INCI: Disodium Cocoyl Glutamate) | 3,80% |
| Mikroemulsion ME9 | 2,00% |
| Perfume | 0,30% |
| Water | 64,30% |
| TEGO® Betain F 50, Evonik Goldschmidt GmbH, 38%-ig, (INCI: Cocamidopropyl Betaine) | 10,00% |
| VARISOFT® PATC, Evonik Goldschmidt GmbH, (INCI: Palmitamidopropyltrimonium Chloride) | 2,30% |
| ANTIL® SPA 80, Evonik Goldschmidt GmbH, (INCI: Isostearamide MIPA, Glyceryl Laurate) | 2,00% |
| Preservative | 0,30% |
| Citric Acid, 30 %-ig | q.s. |

**Formulierungsbeispiel 3) Clear Conditioning Shampoo**

| | |
|---|---|
| TEXAPON® NSO, Cognis, 28%-ig (INCI: Sodium Laureth Sulfate) | 32,00% |
| ANTIL® 200, Evonik Goldschmidt GmbH (INCI: PEG-200 Hydrogenated Glyceryl Palmate; PEG-7 Glyceryl Cocoate) | 2,00% |
| Mikroemulsion ME18 | 2,00% |
| Perfume | 0,25% |
| Water | 55,25% |
| Polymer JR 400, Amerchol (INCI: Polyquaternium-10) | 0,20% |
| TEGO® Betain F 50, Evonik Goldschmidt GmbH, 38%-ig (INCI: Cocamidopropyl Betaine) | 8,00% |
| NaCl | 0,30% |
| Preservative | q.s. |

**Formulierungsbeispiel 4) Clear Conditioning Shampoo**

| | |
|---|---|
| TEXAPON® NSO, Cognis, 28%-ig (INCI: Sodium Laureth Sulfate) | 32,00% |
| ANTIL® 200, Evonik Goldschmidt GmbH (INCI: PEG-200 Hydrogenated Glyceryl Palmate; PEG-7 Glyceryl Cocoate) | 2,00% |
| ABIL® Quat 3272, Evonik Goldschmidt GmbH (INCI: Quaternium-80) | 0,75% |
| Mikroemulsion ME12 | 1,50% |
| Perfume | 0,25% |
| Water | 55,00% |
| Polymer JR 400, Amerchol (INCI: Polyquaternium-10) | 0,20% |
| TEGO® Betain F 50, Evonik Goldschmidt GmbH, 38%-ig (INCI: Cocamidopropyl Betaine) | 8,00% |
| NaCl | 0,30% |
| Preservative | q.s. |

**Formulierungsbeispiel 5) Clear Conditioning Shampoo**

| | |
|---|---|
| TEXAPON® NSO, Cognis, 28%-ig (INCI: Sodium Laureth Sulfate) | 32,00% |
| ANTIL® 200, Evonik Goldschmidt GmbH (INCI: PEG-200 Hydrogenated Glyceryl Palmate; PEG-7 Glyceryl Cocoate) | 2,00% |
| ABIL® B 8832, Evonik Goldschmidt GmbH (INCI: Bis-PEG/PPG-20/20 Dimethicone) | 0,50% |
| Mikroemulsion ME18 | 3,50% |
| Perfume | 0,25% |
| Water | 53,25% |
| Polymer JR 400, Amerchol (INCI: Polyquaternium-10) | 0,20% |
| TEGO® Betain F 50, Evonik Goldschmidt GmbH, 38%-ig (INCI: Cocamidopropyl Betaine) | 8,00% |
| NaCl | 0,30% |
| Preservative | q.s. |

**Formulierungsbeispiel 6) Clear Conditioning Shampoo**

| | |
|---|---|
| TEXAPON® NSO, Cognis, 28%-ig (INCI: Sodium Laureth Sulfate) | 32,00% |
| VARISOFT® PATC, Evonik Goldschmidt GmbH (INCI: Palmitamidopropyltrimonium Chloride) | 1,50% |
| REWODERM® LI S 80, Evonik Goldschmidt GmbH (INCI: PEG-200 Hydrogenated Glyceryl Palmate; PEG-7 Glyceryl Cocoate) | 2,00% |
| Mikroemulsion ME28 | 2,50% |
| Perfume | 0,25% |
| Water | 52,05% |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,20% |
| TEGO® Betain F 50, Evonik Goldschmidt GmbH, 38%-ig (INCI: Cocamidopropyl Betaine) | 8,00% |
| NaCl | 0,50% |
| Preservative | q.s. |

**Formulierungsbeispiel 7) Clear Conditioning Shampoo**

| | |
|---|---|
| TEXAPON® NSO, Cognis, 28%-ig (INCI: Sodium Laureth Sulfate) | 32,00% |
| REWODERM® LI S 80, Evonik Goldschmidt GmbH (INCI: PEG-200 Hydrogenated Glyceryl Palmate; PEG-7 Glyceryl Cocoate) | 2,00% |
| Mikroemulsion ME18 | 2,50% |
| Perfume | 0,25% |
| Water | 53,55% |
| TEGO® Cosmo C 100, Evonik Goldschmidt GmbH, (INCI: Creatine) | 1,00% |
| Jaguar C-162, Rhodia (INCI: Hydroxypropyl Guar Hydroxypropyltrimonium Chloride) | 0,20% |
| TEGO® Betain F 50, Evonik Goldschmidt GmbH, 38%-ig (INCI: Cocamidopropyl Betaine) | 8,00% |
| NaCl | 0,50% |
| Preservative | q.s. |

**Formulierungsbeispiel 8) Pearlized Shampoo**

| | |
|---|---|
| TEXAPON® NSO, Cognis, 28%-ig (INCI: Sodium Laureth Sulfate) | 32,00% |
| Mikroemulsion ME6 | 5,50% |
| Perfume | 0,25% |
| Water | 49,25% |
| TEGO® Betain F 50, Evonik Goldschmidt GmbH, 38%-ig (INCI: Cocamidopropyl Betaine) | 8,00% |
| TEGO® Pearl N 300 Evonik Goldschmidt GmbH (INCI: Glycol Distearate; Laureth-4; Cocamidopropyl Betaine) | 2,00% |
| ANTIL® 171 Evonik Goldschmidt GmbH (INCI: PEG-18 Glyceryl Oleate/Cocoate) | 2,50% |
| NaCl | 0,50% |
| Preservative | q.s. |

**Formulierungsbeispiel 9) Shampoo, PEG- & sulfate free**

| | | |
|---|---|---|
| A | REWOTERIC® AMC, Evonik Goldschmidt GmbH, 32%-ig, (INCI: Sodium Cocoamphoacetate) | 20,00% |
| | REWOPOL® SB F 12 P, Evonik Goldschmidt, 96%-ig, (INCI: Disodium Lauryl Sulfosuccinate) | 5,90% |
| | Mikroemulsion ME18 | 2,00% |
| | ANTIL® SPA 80, Evonik Goldschmidt GmbH, (INCI: Isostearamide MIPA, Glyceryl Laurate) | 1,70% |
| B | Water | 63,20% |
| | Citric Acid, 30%-ig | 3,60% |
| C | ANTIL® HS 60, Evonik Goldschmidt GmbH, (INCI: Cocamidopropyl Betaine; Glyceryl Laurate) | 3,00% |
| | Preservative | 0,60% |

**Formulierungsbeispiel 10) Rinse-Off Conditioner**

| | |
|---|---|
| Water | 85,50% |
| VARISOFT® BT 85, Evonik Goldschmidt GmbH (INCI: Behentrimonium Chloride) | 3,00% |
| Mikroemulsion ME9 | 5,50% |
| TEGO® Alkanol 1618, Evonik Goldschmidt GmbH (INCI: Cetearyl Alcohol) | 5,00% |
| Preservative, Perfume | q.s. |

**Formulierungsbeispiel 11) Rinse-Off Conditioner**

| | |
|---|---|
| Water | 90,20% |
| VARISOFT® EQ 65, Evonik Goldschmidt GmbH (INCI: Distearyl Dimonium Chloride, Cetearyl Alcohol) | 2,00% |
| VARISOFT® BT 85, Evonik Goldschmidt GmbH (INCI: Behentrimonium Chloride) | 1,00% |
| Mikroemulsion ME18 | 1,80% |
| TEGO® Alkanol 1618, Evonik Goldschmidt GmbH (INCI: Cetearyl Alcohol) | 5,00% |
| Preservative, Perfume | q.s. |

**Formulierungsbeispiel 12) Rinse-Off Conditioner**

| | |
|---|---|
| Water | 87,20% |
| VARISOFT® EQ 65, Evonik Goldschmidt GmbH (INCI: Distearyl Dimonium Chloride, Cetearyl Alcohol) | 2,00% |
| VARISOFT® BT 85, Evonik Goldschmidt GmbH (INCI: Behentrimonium Chloride) | 2,00% |
| ABIL® Quat 3272, Evonik Goldschmidt GmbH (INCI: Quaternium-80) | 0,50% |
| Mikroemulsion ME18 | 3,30% |
| TEGO® Alkanol 1618, Evonik Goldschmidt GmbH (INCI: Cetearyl Alcohol) | 5,00% |
| Preservative, Perfume | q.s. |

**Formulierungsbeispiel 13) Rinse-Off Conditioner**

| | |
|---|---|
| TEGINACID® C, Evonik Goldschmidt GmbH (INCI: Ceteareth-25) | 0,50% |
| TEGO® Alkanol 16, Evonik Goldschmidt GmbH (INCI: Cetyl Alcohol) | 2,00% |
| TEGO® Amid S 18, Evonik Goldschmidt GmbH (INCI: Stearamidopropyl Dimethylamine) | 1,00% |
| Mikroemulsion ME28 | 5,50% |
| Propylene Glycol | 2,00% |
| Citric Acid Monohydrate | 0,30% |
| Water | 88,70% |
| Preservative, Perfume | q.s. |

**Formulierungsbeispiel 14) Rinse-Off Conditioner**

| | |
|---|---|
| TEGINACID® C, Evonik Goldschmidt GmbH (INCI: Ceteareth-25) | 0,50% |
| TEGO® Alkanol 16, Evonik Goldschmidt GmbH (INCI: Cetyl Alcohol) | 5,00% |
| TEGOSOFT® DEC, Evonik Goldschmidt GmbH (INCI: Diethylhexyl Carbonate) | 1,00% |
| Mikroemulsion ME18 | 3,50% |
| Water | 87,20% |
| TEGO® Cosmo C 100 Evonik Goldschmidt GmbH (INCI: Creatine) | 0,50% |
| Propylene Glycol | 2,00% |
| Citric Acid Monohydrate | 0,30% |
| Preservative, Perfume | q.s. |

**Formulierungsbeispiel 15) Leave-In Conditioner Spray**

| | |
|---|---|
| Lactic Acid, 80% | 0,40% |
| Water | 92,30% |
| TEGO® Amid S 18, Evonik Goldschmidt GmbH (INCI: Stearamidopropyl Dimethylamine) | 1,20% |
| TEGIN® G 1100 Pellets, Evonik Goldschmidt GmbH (INCI: Glycol Distearate) | 0,60% |
| TEGO® Care PS, Evonik Goldschmidt GmbH (INCI: Methyl Glucose Sesquistearate) | 1,20% |
| TEGOSOFT® DEC, Evonik Goldschmidt GmbH (INCI: Diethylhexyl Carbonate) | 0,30% |
| Mikroemulsion ME6 | 4,00% |
| Preservative, Perfume | q.s. |

**Formulierungsbeispiel 16) Leave-In Conditioner Spray**

| | |
|---|---|
| TAGAT® CH-40, Evonik Goldschmidt GmbH (INCI: PEG-40 Hydrogenated Castor Oil) | 2,00% |
| Ceramide VI, Evonik Goldschmidt GmbH (INCI: Ceramide 6 II) | 0,05% |
| Perfume | 0,20% |
| Water | 81,95% |
| Mikroemulsion ME6 | 9,50% |
| LACTIL® Evonik Goldschmidt GmbH (INCI: Sodium Lactate; Sodium PCA; Glycine; Fructose; Urea; Niacinamide; Inositol; Sodium benzoate; Lactic Acid) | 2,00% |
| TEGO® Betain F 50 Evonik Goldschmidt GmbH 38% (INCI: Cocamidopropyl Betaine) | 2,30% |
| Citric Acid (10% in water) | 2,00% |

**Formulierungsbeispiel 17) Leave-In Conditioner Foam**

| | |
|---|---|
| Mikroemulsion ME18 | 3,50% |
| TAGAT® CH-40, Evonik Goldschmidt GmbH (INCI: PEG-40 Hydrogenated Castor Oil) | 0,50% |
| Perfume | 0,30% |
| TEGO® Betain 810, Evonik Goldschmidt GmbH (INCI: Capryl/Capramidopropyl Betaine) | 2,00% |
| Water | 91,00% |
| TEGO® Cosmo C 100, Evonik Goldschmidt GmbH (INCI: Creatine) | 0,50% |
| TEGOCEL® HPM 50, Evonik Goldschmidt GmbH (INCI: Hydroxypropyl Methylcellulose) | 0,30% |
| VARISOFT® 300, Evonik Goldschmidt GmbH (INCI: Cetrimonium Chloride) | 1,30% |
| LACTIL® Evonik Goldschmidt GmbH (INCI: Sodium Lactate; Sodium PCA; Glycine; Fructose; Urea; Niacinamide; Inositol; Sodium benzoate; Lactic Acid) | 0,50% |
| Citric Acid (30% in water) | 0,10% |
| Preservative | q.s. |

**Formulierungsbeispiel 18) Strong Hold Styling Gel**

| | |
|---|---|
| TEGO® Carbomer 141, Evonik Goldschmidt GmbH (INCI: Carbomer) | 1,20% |
| Water | 65,00% |
| NaOH, 25% | 2,70% |
| PVP/VA W-735, ISP (INCI: PVP/VA Copolymer) | 16,00% |
| Mikroemulsion ME18 | 2,50% |
| Alcohol Denat. | 10,00% |
| TAGAT® O 2 V, Evonik Goldschmidt GmbH (INCI: PEG-20 Glyceryl Oleate) | 2,00% |
| Perfume | 0,30% |
| ABIL® B 88183, Evonik Goldschmidt GmbH (INCI: PEG/PPG-20/6 Dimethicone) | 0,30% |
| Preservative | q.s. |

**Formulierungsbeispiel 19) Schaumiges Körperpflegemittel**

| | |
|---|---|
| TEXAPON® NSO, Cognis, 28%-ig (INCI: Sodium Laureth Sulfate) | 14,30% |
| Perfume | 0,30% |
| Mikroemulsion ME18 | 1,50% |
| REWOTERIC® AM C, Evonik Goldschmidt GmbH, 32%-ig (INCI: Sodium Cocoamphoacetate) | 8,00% |
| Water | 73,90% |
| TEGOCEL® HPM 50, Evonik Goldschmidt GmbH (INCI: Hydroxypropyl Methylcellulose) | 0,50% |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 1,00% |
| Citric Acid Monohydrate | 0,50% |

**Formulierungsbeispiel 20) Körperpflegemittel**

| | |
|---|---|
| TEXAPON® NSO, Cognis, 28%-ig (INCI: Sodium Laureth Sulfate) | 30,00% |
| TEGOSOFT® PC 31, Evonik Goldschmidt GmbH (INCI: Polyglyceryl-3 Caprate) | 0,50% |
| Mikroemulsion ME12 | 1,50% |
| Perfume | 0,30% |
| Water | 52,90% |
| TEGOCEL® HPM 4000, Evonik Goldschmidt GmbH (INCI: Hydroxypropyl Methylcellulose) | 0,30% |
| REWOTERIC® AM C, Evonik Goldschmidt GmbH, 32%-ig (INCI: Sodium Cocoamphoacetate) | 10,00% |
| Citric Acid Monohydrate | 0,50% |
| REWODERM® LI S 80, Evonik Goldschmidt GmbH (INCI: PEG-200 Hydrogenated Glyceryl Palmate; PEG-7 Glyceryl Cocoate) | 2,00% |
| TEGO® Pearl N 300, Evonik Goldschmidt GmbH (INCI: Glycol Distearate; Laureth-4; Cocamidopropyl Betaine) | 2,00% |

**Formulierungsbeispiel 21) Schaumiges Körperpflegemittel**

| | |
|---|---|
| TEXAPON® NSO, Cognis, 28%-ig (INCI: Sodium Laureth Sulfate) | 14,30% |
| Perfume | 0,30% |
| Mikroemulsion ME18 | 1,o0% |
| REWOTERIC® AM C, Evonik Goldschmidt GmbH, 32%-ig (INCI: Sodium Cocoamphoacetate) | 8,00% |
| Water | 75,10% |
| Polyquaternium-7 | 0,30% |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 0,50% |
| Citric Acid Monohydrate | 0,50% |

**Formulierungsbeispiel 22) Mild Foam Bath**

| | |
|---|---|
| TEXAPON® NSO, Cognis, 28%-ig (INCI: Sodium Laureth Sulfate) | 27,00% |
| REWOPOL® SB FA 30, Evonik Goldschmidt GmbH, 40%-ig (INCI: Disodium Laureth Sulfosuccinate) | 12,00% |
| TEGOSOFT® LSE 65 K SOFT, Evonik Goldschmidt GmbH (INCI: Sucrose Cocoate) | 2,00% |
| Water | 38,00% |
| REWOTERIC® AM C, Evonik Goldschmidt GmbH, 32%-ig (INCI: Sodium Cocoamphoacetate) | 13,00% |
| Mikroemulsion ME7 | 1,50% |
| Citric Acid (30% in water) | 3,00% |
| ANTIL® 171 Evonik Goldschmidt GmbH (INCI: PEG-18 Glyceryl Oleate/Cocoate) | 1,50% |
| TEGO® Pearl N 300 Evonik Goldschmidt GmbH (INCI: Glycol Distearate; Laureth-4; Cocamidopropyl Betaine) | 2,00% |

**Formulierungsbeispiel 23) Rinse-Off Conditioner**

| | |
|---|---|
| Water | 88,20% |
| VARISOFT® 300, Evonik Goldschmidt GmbH (INCI: Cetrimonium Chloride) | 2,00% |
| VARISOFT® BT 85, Evonik Goldschmidt GmbH (INCI: Behentrimonium Chloride) | 2,00% |
| ABIL® OSW 5, Evonik Goldschmidt GmbH (INCI: Cyclopentasiloxane; Dimethiconol) | 1,00% |
| Mikroemulsion ME6 | 1,80% |
| TEGO® Alkanol 1618, Evonik Goldschmidt GmbH (INCI: Cetearyl Alcohol) | 5,00% |
| Preservative, Perfume | q.s. |

**Formulierungsbeispiel 24) Rinse-Off Conditioner**

| | |
|---|---|
| Water | 87,20% |
| VARISOFT® EQ 65, Evonik Goldschmidt GmbH (INCI: Distearyl Dimonium Chloride, Cetearyl Alcohol) | 2,00% |
| VARISOFT® BT 85, Evonik Goldschmidt GmbH (INCI: Behentrimonium Chloride) | 2,00% |
| ABIL® Soft AF 100, Evonik Goldschmidt GmbH (INCI: Methoxy PEG/PPG-7/3 Aminopropyl Dimethicone) | 1,00% |
| Mikroemulsion ME18 | 2,80% |
| TEGO® Alkanol 1618, Evonik Goldschmidt GmbH (INCI: Cetearyl Alcohol) | 5,00% |
| Preservative, Perfume | q.s. |

**Formulierungsbeispiel 25) Rinse-Off Conditioner**

| | |
|---|---|
| Water | 88,20% |
| VARISOFT® BT 85, Evonik Goldschmidt GmbH (INCI: Behentrimonium Chloride) | 3,00% |
| SF 1708, Momentive (INCI: Amodimethicone) | 2,00% |
| Mikroemulsion ME18 | 1,80% |
| TEGO® Alkanol 1618, Evonik Goldschmidt GmbH (INCI: Cetearyl Alcohol) | 5,00% |
| Preservative, Perfume | q.s. |

**Formulierungsbeispiel 26) feuchtigkeitspendendes Hautreinigungsmittel**

| | | |
|---|---|---|
| A | TEXAPON® NSO, Cognis, 28%-ig, (INCI: Sodium Laureth Sulfate) | 30,00 |
| | Mikroemulsion ME12 | 1,70 |
| | Perfume | 0,30 |
| B | Water | 54,60 |
| | TEGOCEL® fluid HPM 4000, Evonik Goldschmidt GmbH, (INCI: Hydroxypropyl Methylcellulose) | 1,20 |
| | TEGO® Betain C 60, Evonik Goldschmidt GmbH, 46%-ig, (INCI: Cocamidopropyl Betaine) | 8,10 |
| | TEGOSOFT® APM, Evonik Goldschmidt GmbH, (INCI: PPG-3 Myristyl Ether) | 1,00 |
| | Cutina TS, Cognis (INCI:PEG- 3 Distearate) | 1,00 |
| | REWODERM® LI S 80, Evonik Goldschmidt GmbH, (INCI: PEG-200 Hydrogenated Glyceryl Palmate; PEG-7 Glyceryl Cocoate) | 1,50 |
| | Preservative | 0,60 |
| | Citric Acid, 30%-ig | q.s. |

**Formulierungsbeispiel 27) Duschgel**

| | |
|---|---|
| TEXAPON® NSO, Cognis, 28%-ig (INCI: Sodium Laureth Sulfate) | 15,00 |
| Mikroemulsion ME28 | 1,50 |
| Perfume | 0,30 |
| PGFAC-S, Cognis (INCI: Sodium cocoyl hydrolyzed wheat protein glutamate) | 1,50 |
| REWOPOL SB CS 50 B, Evonik Goldschmidt GmbH, 40%-ig, (INCI: Disodium PEG-5 Laurylcitrate Sulfosuccinate; Sodium Laureth Sulfate) | 7,50 |
| Water | 58,10 |
| TEGO® Betain F 50, Evonik Goldschmidt GmbH, 38%-ig, (INCI: Cocamidopropyl Betaine) | 9,00 |
| TEGO® Betain 810, Evonik Goldschmidt GmbH, 38%-ig, (INCI: Capryl/Capramidopropyl Betaine) | 4,00 |
| Polyquaternium-7, Nalco, (INCI: Merquat 550) | 0,50 |
| ANTIL® 200, Evonik Goldschmidt GmbH, (INCI: PEG-200 Hydrogenated Glyceryl Palmate; PEG-7 Glyceryl Cocoate) | 2,30 |
| Preservative | 0,30 |

**Formulierungsbeispiel 28) Körperreinigungsmittel**

| | | |
|---|---|---|
| A | TEXAPON® NSO Cognis 28%-ig,(INCI: Sodium Laureth Sulfate | 30,00 |
| | Mikroemulsion ME18 | 1,50 |
| | ABIL® B 8832, Evonik Goldschmidt GmbH, (INCI: Bis-PEG/PPG-20/20 Dimethicone) | 0,30 |
| | Perfume | 0,30 |
| B | Water | 51,00 |
| | TEGOCEL® fluid HPM 4000, Evonik Goldschmidt GmbH, (INCI: Hydroxypropyl Methylcellulose) | 1,20 |
| | Citric Acid Monohydrate | 0,50 |
| | REWOTERIC® AM C, Evonik Goldschmidt GmbH, 32%-ig, (INCI: Sodium Cocoamphoacetate) | 10,00 |
| | Cutina TS, Cognis (INCI:PEG- 3 Distearate) | 2,00 |
| | REWODERM® LI S 80, Evonik Goldschmidt GmbH, (INCI: PEG-200 Hydrogenated Glyceryl Palmate; PEG-7 Glyceryl Cocoate) | 2,60 |
| | Preservative | 0,60 |
| | Citric Acid, 30%-ig | q.s. |

**Formulierungsbeispiel 29) Body cleansing Foam**

| | |
|---|---|
| TEXAPON® NSO, Cognis, 28%-ig (INCI: Sodium Laureth Sulfate) | 14 |
| Perfume | 0,3 |
| Mikroemulsion ME18 | 0,7 |
| REWOTERIC® AM C, Evonik Goldschmidt GmbH, 32%-ig (INCI: Sodium Cocoamphoacetate) | 8 |
| Water | 74,8 |
| TEGOCEL® HPM 50, Evonik Goldschmidt GmbH (INCI: Hydroxypropyl Methylcellulose) | 0,5 |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 1 |
| Panthenol, BASF, (INCI: D-Panthenol USP) | 0,2 |
| Citric Acid Monohydrate | 0,5 |

**Formulierungsbeispiel 30) Turbid Conditioning Shampoo**

| | |
|---|---|
| TEXAPON® NSO, Cognis, 28%-ig (INCI: Sodium Laureth Sulfate) | 32,00 |
| ANTIL® 200, Evonik Goldschmidt GmbH (INCI: PEG-200 Hydrogenated Glyceryl Palmate; PEG-7 Glyceryl Cocoate) | 2,00 |
| Mikroemulsion ME12 | 1,00 |
| Perfume | 0,25 |
| Water | 53,25 |
| Polymer JR 400, Amerchol (INCI: Polyquaternium-10) | 0,20 |
| TEGO® Betain F 50, Evonik Goldschmidt GmbH, 38%-ig (INCI: Cocamidopropyl Betaine) | 8,00 |
| DC1503 Fluid, Dow Corning, (INCI: Dimethicone, Dimethiconol) | 1,00 |
| TEGO® Pearl N 300 Evonik Goldschmidt GmbH (INCI: Glycol Distearate; Laureth-4; Cocamidopropyl Betaine) | 2,00 |
| NaCl | 0,30 |
| Preservative | q.s. |

**Formulierungsbeispiel 31) Mild Hair & Body Wash, PEG- und Sulfate-free**

| | |
|---|---|
| Plantacare® 1200 UP, Cognis, 50%-ig, (INCI: Lauryl Glucoside) | 11,40% |
| Plantacare® 818 UP, Cognis, 51%-ig, (INCI: Coco Glucoside) | 5,60% |
| Water | 61,60% |
| ANTIL® SOFT SC, Evonik Goldschmidt GmbH, (INCI: Sorbitan Sesquicaprylate) | 0,90% |
| Mikroemulsion ME28 | 1,00% |
| TEGOSOFT® LSE 65 K SOFT, Evonik Goldschmidt GmbH, (INCI: Sucrose Cocoate) | 1,50% |
| TEGO® Betain F 50, Evonik Goldschmidt GmbH, 38%-ig, (INCI: Cocamidopropyl Betaine) | 18,00% |
| Perfume, preservative | q.s. |
| Citric Acid, 30% | q.s. |

**Formulierungsbeispiel 32) Sprayable Hairmilk, PEG-free**

| | | |
|---|---|---|
| A | Water | 95,30% |
| | Lactic Acid, 80%-ig | 0,40 |
| B | TEGO® AMID S 18, Evonik Goldschmidt GmbH, (INCI: Stearamidopropyl Dimethylamine) | 1,20% |
| | TEGIN® G 1100 Pellets, Evonik Goldschmidt GmbH, (INCI: Glycol Distearate) | 0,60% |
| | TEGO® Care PS, Evonik Goldschmidt GmbH, (INCI: Methyl Glucose Sesquistearate) | 1,20% |
| | TEGOSOFT® DEC, Evonik Goldschmidt GmbH, (INCI: Diethylhexyl Carbonate) | 0,30% |
| | Mikroemulsion ME18 | 1,00% |
| | Perfume, preservative | q.s. |

### Anwendungstechnik Textilpflege

Zur Bestimmung der weichmachenden Wirkung der erfindungsgemäßen Mikroemulsion auf textilen Geweben wurden Baumwolltücher damit behandelt.

### Herstellung einer Siloxan-Makroemulsion (nicht erfindungsgemäß - Vergleichsbeispiel) (Formulierungbeispiel T1)

20 Teile des auf 40°C bis 80°C erwärmten Siloxans 5 werden in einem Becherglas mit Propellerrührer unter Rühren vorgelegt. Anschließend werden der Reihe nach 10 Gew.-Teile Dipropylenglykol, 10 Gew.-Teile eines Fettalkoholethoxylates mit einem Ethoxylierungsgrad von 6 unter Rühren hinzugegeben. Zuletzt wird mit Wasser auf 100 Gew.-Teile aufgefüllt und solange nachgerührt, bis die Mischung auf Raumtemperatur abgekühlt ist, mindestens jedoch 15 min.

### Herstellung einer Siloxan-Mikroemulsion (erfindungsgemäß) Formulierungsbeispiel T2: Entspricht dem Formulierungsbeispiel Mikroemulsion ME 33

### Herstellung von Weichspülerformulierungen:

Formulierungsbeispiel T3: ca. 5 Gew.-% Siloxanfreie Weichspülerformulierung (Nicht erfindungsgemäß - Referenz)
33,3 g eines auf 40 bis 80°C erwärmten flüssigen REWOQUAT® WE 18 (Handelsname der Evonik Goldschmidt GmbH, Triethanolamin basiertes Esterquat mit einem Aktivgehalt von 90%) wurden unter Rühren zu 556 g auf 45 - 65 °C erwärmten Leitungswasser gegeben, 20 min mit einem Propellerrührer bei 45 - 65°C gerührt und innerhalb von ca. einer Stunde auf Raumtemperatur abgekühlt.

### Formulierungsbeispiel T4: Siloxanhaltige Weichspülerformulierung (Nicht erfindungsgemäß - Vergleichsbeispiel)

33,3 g eines auf 40 bis 80°C erwärmten flüssigen REWOQUAT® WE 18 wurden in einem 50 ml Zentrifugenröhrchen aus Polypropylen zusammen mit 0,28 g des Siloxans 5 eingewogen und mit einem Vortexmischer durch Schütteln intensiv gemischt. Dieses trübe Gemisch wurden unter Rühren vollständig zu 556 g auf 45 - 65 °C erwärmten Leitungswasser gegeben, 20 min mit einem Propellerrührer bei 45 - 65°C gerührt und innerhalb von ca. einer Stunde auf Raumtemperatur abgekühlt.

### Formulierungsbeispiel T5: Siloxanhaltige Weichspülerformulierung (Nicht erfindungsgemäß - Vergleichsbeispiel)

33,3 g eines auf 40 bis 80°C erwärmten flüssigen REWOQUAT® WE 18 (Handelname der Evonik Goldschmidt GmbH, Triethanolamin basiertes Esterquat mit einem Aktivgehalt von 90%) wurden unter Rühren zu 556 g auf 45 - 65 °C erwärmten Leitungswasser gegeben, 20 min mit einem Propellerrührer bei 45 - 65°C gerührt, anschließend wurden 1,40 g der Siloxan-Makroemulsion aus Formulierungsbeispiel T2 hinzugegen und innerhalb von ca. einer Stunde auf Raumtemperatur abgekühlt

### Formulierungsbeispiel T6: Siloxanhaltige Weichspülerformulierung (erfindungsgemäß):

33,3 g eines auf 40 bis 80°C erwärmten flüssigen REWOQUAT® WE 18 (Handelname der Evonik Goldschmidt GmbH, Triethanolamin basiertes Esterquat mit einem Aktivgehalt von 90%) wurden unter Rühren zu 556 g auf 45 - 65 °C erwärmten Leitungswasser gegeben, 20 min mit einem Propellerrührer bei 45 - 65°C gerührt, anschließend wurden 1,37 g der Siloxan Mikroemulsion aus Formulierungsbeispiel T3 hinzugeben und innerhalb von ca. einer Stunde auf Raumtemperatur abgekühlt

### Vorbehandlung des Baumwollgewebes:

Baumwollfrottiergewebe von 80 cm x 50 cm mit einem Flächengewicht von ca. 350 g/m² wurden zweimal mit Vollwaschpulver gewaschen, zweimal gespült, geschleudert und einlagig auf einer Leine hängend an der Luft getrocknet.

### Behandlung von Baumwollgewebe:

Die oben beschriebene Formulierungen T3 bis T6 wurden mit kaltem Leitungswasser zu Spüllösungen verdünnt, deren Gesamtaktivkonzentration, das ist die Summe aus REWOQUAT® WE 18 und Siloxan 5, 0,025 Gew.-% beträgt.

Die Baumwolltücher wurden 10 min in zwei Liter der Spüllösung getaucht. Dabei ist zu beachten, dass die Tücher gleichmäßig von der Spüllösung benetzt werden. Anschließend wurden die Tücher geschleudert und bei Raumtemperatur einlagig auf einer Leine hängend getrocknet. Die behandelten Baumwollfrottiertücher wurden in 10 gleiche Stücke von 16 cm auf 25 cm geschnitten.

Zur Beurteilung des Weichgriffes wurde ein erfahrenes Team aus 9 Testpersonen zusammengestellt, das die anonymisierten Griffmuster, der mit den Spüllösungen behandelten Baumwollgewebe, mit Hilfe eines Handpaneltests bewertete. Dabei erhält jede Testperson ein eignes Baumwolltuch. Die Beurteilung erfolgt dabei auf einer Skala von 0 (hart und unangenehm im Griff) bis 5 (weich und angenehm im Griff) mit der Möglichkeit zu ganzzahligen Zwischenwerten.
Zur Beurteilung des Weichgriffes werden die Einzelbewertungen summiert, so dass sich bei 9 Testpersonen ein maximaler Weichgriff von 45 ergeben kann.

Bei den Griffmustern wurde zusätzlich immer eine nicht offensichtlich gekennzeichnete unbehandelte Probe(Blindwert) hinzugelegt.

### Zusammenfassung der Weichgriffergebnisse

| Formulierungsbeispiel | Bemerkung | Weichgriff |
|---|---|---|
| T3 | ohne Siloxan 5 | 33 |
| T4 | Siloxan 5 gemischt mit REWOQUAT® WE 18 | 29 |
| T5 | Makroemulsion von Siloxan 5 | 32 |
| T6 | Mikroemulsion von Siloxan 5 | 39 |
| Blindwert | ohne Siloxan & ohne REWOQUAT® WE 18 | 0 |

Aus den Daten in Tabelle ist deutlich zu ersehen, dass die Weichheit des Baumwollgewebes beim Einsatz einer Mikroemulsion von Siloxan 5 stark verbessert werden kann. Die direkte Mischung von REWOQUAT® WE 18 und Siloxan 5, sowie der Einsatz einer Makroemulsion führen hingegen zu einer Verringerung des Weichgriffes.

### Anwendungstechnik Beschichtungen:

**Formulierungsbeispiel 1) 1K PUR-Lack**

| | |
|---|---|
| Bayderm Finish 91 UD | 54,0% |
| Wasser | 45,0% |
| TEGO® Viskoplus 3030 | 1,0% |

### Herstellung von Beschichtungen:

100 Gram 1K PUR-Lack zusammen mit entweder 0,0, 1,5 oder 3,0 Gram von der Mikroemulsion ME35 oder ME36 wurden in einem 180 mL Polyethylen-Becher (Ø 6 cm) abgewogen und anschließend mit einem Dispermaten mit einer Zahnscheibe (Ø 3 cm) durch 3 Minuten Rühren bei 2000 Upm homogenisiert (Probe 1-5). Nach 24 Stunden Verweilzeit wird der Lack auf schwarzer, mattierter PVC Folie (System Leneta ®, 43 x 28 cm) mit Hilfe eines 60 µm Kastenrakels appliziert. Anschließend wird die Beschichtung über 72 Stunden bei Raumtemperatur getrocknet. Alternativ wird der Lack mit Hilfe eines 75 µm Spiralrakels auf beiges Rindsleder appliziert und ebenfalls getrocknet.

### Ausprüfung (Haft)Gleitreibung (Gleitwert):

Die Gleitwerte wurden auf PVC-Folie mit einem Instron 3300 Gerät (Instron Deutschland GmbH, Pfungstadt, Deutschland) gemessen. Das Gerät misst die Kraft, die nötig ist, um einen Schlitten über die beschichtete Folie zu ziehen. Der zylinderförmige Schlitten von 500 Gram Gewicht und eine Kontaktoberfläche von 12,6 cm² wurde dabei mit einer Filz-Oberfläche ausgestattet, wobei diese Oberfläche für jede Messung erneuert wird. Anschießend wird so lange (mit zunehmender Kraft) an dem Schlitten gezogen bis er sich mit einer Geschwindigkeit von 6 mm/s bewegt. Es wird also die Zugkraft (Last) gemessen, die notwendig ist um den Schlitten zu bewegen. Eine hohe Haftgleitreibung oder Stick-Slip-Verhalten macht sich durch starke Schwankungen der Zugkraft (Last) in der Messung merkbar.

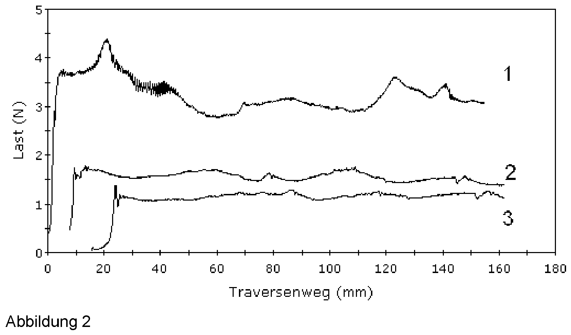

Abbildung 2 zeigt einige repräsentative Messkurven. Mit zunehmender Konzentration der Mikroemulsion nimmt die Gleitreibung ab. Die starken Schwankungen der Last bei der nicht-additivierten Probe deuten auf eine große Haftgleitreibung hin. Durch Zugabe von ME35 werden die Schwankungen der Last in der Messung verringert, d.h. die Haftgleitreibung kleiner.

### Ausprüfung Griff:

Zur Beurteilung der Haptik hat eine erfahrene Testperson die auf Rindsleder aufgezogenen Beschichtungen qualitativ bewertet. Testperson bezeichnet Probe 1 als gummiartig. Die Proben 2 bis 5 dagegen werden als samtig empfunden.

### Zusammenfassung der Austestung

| Probe | ME35 / Gram | ME36 / Gram | | Gleitwert cN | | Griff |
|---|---|---|---|---|---|---|
| 1 | 0,0 | 0,0 | | 306 | | Gummi |
| 2 | 1,5 | 0,0 | | 154 | | Samtig |
| 3 | 3,0 | 0,0 | | 126 | | Samtig |
| 4 | 0,0 | 1,5 | | 105 | | Samtig |
| 5 | 0,0 | 3,0 | | 92 | | Samtig |

## Patentansprüche

1. Mikroemulsion als maßgeblich die Öl-Phase bildende Komponente enthaltend A) mindestens eine quaternäre Ammoniumgruppe enthaltendes Polysiloxan der allgemeinen Formel (I)
Mₐ M'ₐ₁ M"ₐ₂ M'"ₐ₃ D_{b} D'_{b1} D"_{b2} D'"_{b3} T_{c} T'_{c1} Qd Formel (I),
wobei
M = (R¹₃ Si O_{1/2})
M' = (R²R¹₂ Si O_{1/2})
M" = (R³R¹₂ Si O_{1/2})
M'" = (R⁴R¹₂ Si O_{1/2})
D = (R¹₂ Si O_{2/2})
D' = (R²R¹ Si O_{2/2})
D" = (R³R¹ Si O_{2/2})
D'" = (R⁴R¹ Si O_{2/2})
T = (R⁵ Si O_{3/2})
T' = (R² Si O_{3/2})
Q = (Si O_{4/2})
a = 0 bis 32; bevorzugt 0 bis 22, insbesondere 0 bis 12;
a1 = 0 bis 10, bevorzugt 0 bis 5, insbesondere 0;
a2 = 0 bis 32; bevorzugt 0 bis 22, insbesondere 1 bis 12;
a3 = 0 bis 10; bevorzugt 0 bis 5, insbesondere 0;
mit der Maßgabe, dass
a + a1 + a2 + a3 ≥ 3, bevorzugt > 3;
b = 20 bis
400;
b1 = 0 bis 10, bevorzugt 0 bis 5, insbesondere 0;
b2 = 0 bis 80, bevorzugt 0 bis 50, insbesondere 0 bis 10;
b3 = 0 bis 20, bevorzugt 0 bis 10, insbesondere 0;
c = 0 bis 30, bevorzugt 1 bis 20, insbesondere 2 bis 15;
c1 = 0 bis 10, bevorzugt 0 bis 5, insbesondere 0;
d = 0 bis 15, bevorzugt 1 bis 12, insbesondere 2 bis 10;
mit der Maßgabe, dass
a2 + b2 ≥ 1, bevorzugt ≥ 3, insbesondere >3 und
c + c1 + d ≥ 1, bevorzugt > 1, insbesondere ≥ 3;
R¹ = Methyl;
R² = unabgängig voneinander gleiche oder verschiedene Alkoxy- oder Acyloxyreste, wie beispielsweise Methoxy-, Ethoxy-, n-Propoxy oder iso-Propoxyreste, Acetoxy, insbesondere Ethoxy oder iso-Propoxyreste;
R³ = unabhängig voneinander gleiche oder verschiedene organische Reste, die quaternäre Ammoniumfunktionen tragen;
R⁴ = unabhängig voneinander gleiche oder verschiedene organische Epoxy-Reste;
R⁵ = unabhängig voneinander gleiche oder verschiedene Reste R¹, R³ oder R⁴, bevorzugt R¹, insbesondere Methyl, Phenyl, Dodecyl oder Hexadecyl
sind.

2. Mikroemulsion gemäß Anspruch 1 **dadurch gekennzeichnet, dass** sie ein Polysiloxan enthält mit
R⁴ gleiche oder verschiedene Reste ausgewählt aus der Gruppe

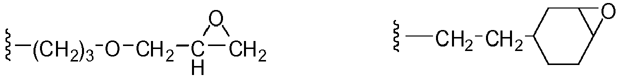

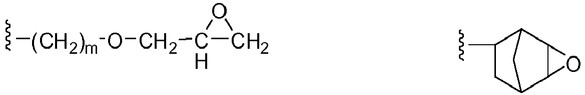

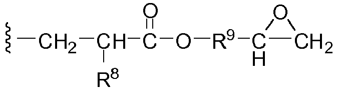

3. Mikroemulsion gemäß Anspruch 1 **dadurch gekennzeichnet, dass** sie ein Polysiloxan enthält mit
R³ Gruppen mit dem Aufbau -R⁶-R⁷, worin
R⁶ vorzugsweise gleiche oder verschiedene zweiwertige Reste sind, ausgewählt aus der Gruppe 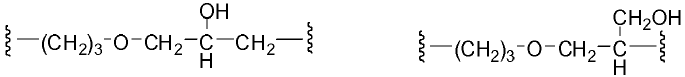 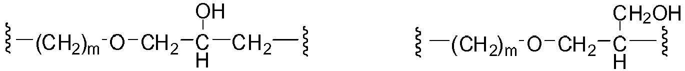 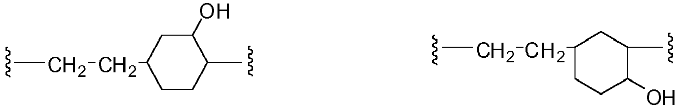 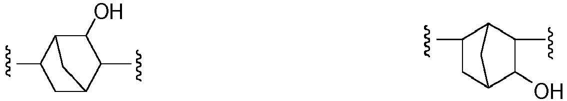 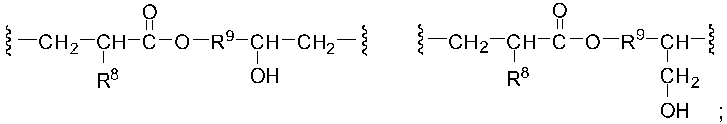
R⁷ ausgewählt ist aus der Gruppe bestehend aus 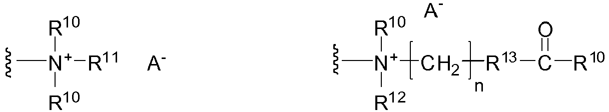 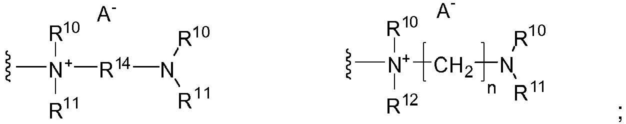
R⁸ sind gleiche oder verschiedene Reste aus der Gruppe Wasserstoff oder Alkyl mit 1 bis 6 C-Atomen, bevorzugt Methyl;
R⁹ sind gleiche oder verschiedene zweiwertige Kohlenwasserstoffreste, die gegebenenfalls Etherfunktionen enthalten, bevorzugt Methylen;
R¹⁰, R¹¹, R¹² sind jeweils unabhängig voneinander Wasserstoff oder Alkylreste mit 1 bis 30 C-Atomen oder Reste der Formel 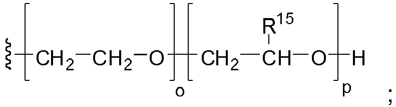
R¹³ sind gleiche oder verschiedene Reste aus der Gruppe -
O-; -NR¹⁶-;
R¹⁴ sind gleiche oder verschiedene gegebenenfalls verzweigte divalente Kohlenwasserstoffreste, bevorzugt Ethylen oder Propylen;
R¹⁵ sind gleiche oder verschiedene Alkyl-, Aryl- oder Alkarylreste mit 1 bis 30 C-Atomen, die gegebenenfalls Etherfunktionen enthalten, bevorzugt Methyl, Ethyl oder Phenyl, insbesondere Methyl;
R¹⁶ sind gleiche oder verschiedene Reste aus der Gruppe Wasserstoff oder Alkyl mit 1 bis 6 C-Atomen;
m = 2 bis 18;
n = 2 bis 18, bevorzugt 3;
o = 0 bis 30, bevorzugt 0 bis 10, insbesondere 1 bis 3;
p = 0 bis 30, bevorzugt 0 bis 10;
A⁻ sind gleiche oder verschiedene Gegenionen zu den positiven Ladungen an den quaternierten Stickstoffgruppen, ausgewählt aus anorganischen oder organischen Anionen der Säuren HA, sowie deren Derivate.

4. Mikroemulsion gemäß mindestens einem der vorherigen Ansprüche, **dadurch gekennzeichnet, dass** sie ein Polysiloxan enthält mit
c = 1 und
c + c1 + d = 1.

5. Mikroemulsion gemäß mindestens einem der vorherigen Ansprüche, **dadurch gekennzeichnet, dass** das Polysiloxan ein mittleres Molekulargewicht aufweist von größer 4000 g/mol, bevorzugt von größer 7000 g/mol, insbesondere von größer 10000 g/mol.

6. Mikroemulsion gemäß mindestens einem der vorherigen Ansprüche, **dadurch gekennzeichnet, dass** sie zusätzlich mindestens eine der Komponenten enthält
B) ein nichtionisches Tensid
C) ein Cotensid ausgewählt aus der Gruppe enthaltend, bevorzugt bestehend aus anionischen, kationischen und amphoteren Tensiden und
D) Wasser.

7. Mikroemulsion gemäß mindestens einem der vorherigen Ansprüche, **dadurch gekennzeichnet, dass** sie im Wesentlichen frei von alkoxylierten Verbindungen ist.

8. Mikroemulsion gemäß Anspruch 7, **dadurch gekennzeichnet, dass** Komponente B) ausgewählt ist aus der Gruppe bestehend aus
Glycerinmono- und -diester und Sorbitanmono- und -diester von gesättigten und ungesättigten Fettsäuren,
Alkylmono- und -oligoglycoside, Partialester auf Basis linearer, verzweigter, ungesättigter bzw. gesättigter Fettsäuren,
Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose),
Mono-, Di- und Trialkylphosphate und deren Salze,
Zitronensäureester wie z.B. Glyceryl Stearate Citrate, Glyceryl Oleate Citrate und Dilauryl Citrate sowie Glyceryl Caprylate, Polyglycerylcaprylate, Polyglycerylcaprate
und Mischungen dieser Tenside.

9. Mikroemulsion gemäß mindestens einem der vorherigen Ansprüche, **dadurch gekennzeichnet, dass** zusätzlich eine Komponente E), Lösungsmittel, enthalten ist ausgewählt aus der Gruppe enthaltend
Hydrotrope beispielsweise aus der Gruppe der aliphatischen Alkohole, wie Ethanol, Propanol oder 1,3-Propandiol, zyklische Carbonate wie Ethylencarbonat, Propylencarbonat, Glycerincarbonat, Ester von Mono- oder Polycarbonsäuren wie Ethylacetat, Ethyllactat, Glycerin, Isopropylalkohol, Dipropylenglykol, Glykolether und Polyole.

10. Mikroemulsion gemäß mindestens einem der vorherigen Ansprüche, **dadurch gekennzeichnet, dass** zusätzlich eine Komponente F), Konservierungsmittel, und/oder eine Komponente G), ein Öl oder Ölgemisch, enthalten.

11. Mikroemulsion gemäß mindestens einem der vorherigen Ansprüche **dadurch gekennzeichnet, dass**
Komponente A) in einer Menge von 10 Gew.-% bis 60 Gew.-%;
Komponente B) in einer Menge von 3 Gew.-% bis 30 Gew.-%;
Komponente C) in einer Menge von 0 Gew.-% bis 30 Gew.-%;
Komponente D) in einer Menge von 10 Gew.-% bis 75 Gew.-%;
Komponente E) in einer Menge von 0 Gew.-% bis 35 Gew.-%;
Komponente F) in einer Menge von 0 Gew.-% bis 1 Gew.-% und
Komponente G) in einer Menge von 0 Gew.-% bis 50 Gew.-% der gesamten Öl-Phase bestehend aus A) und G),
enthalten ist, wobei sich die Gew.-% außer im Falle von Komponente G) auf die Gesamtmikroemulsion beziehen.

12. Verfahren zur Herstellung einer Mikroemulsion gemäß mindestens einem der vorherigen Ansprüche umfassend die Verfahrensschritte
I) Bereitstellen mindestens eines Polysiloxans wie oben beschrieben,
II) gegebenenfalls Lösen des Polysiloxans mit mindestens einem Lösungsmittel E) und/oder einem nichtionischen Tensid der Komponente B) und/oder Öl G)
III) hinzugeben der restlichen die Mikroemulsion bildenden Komponenten, wobei die Komponenten Wasser und Konservierungsmittel zuletzt zugegeben werden.

13. Verfahren gemäß Anspruch 12 **dadurch gekennzeichnet, dass** während der Verfahrensschritte I) bis III) mit einem einfachen Rührer gemischt wird

14. Verwendung mindestens einer Mikroemulsion gemäß mindestens einem der Ansprüche 1 bis 11 oder mindestens eine Mikroemulsion erhältlich nach einem Verfahren gemäß Anspruch 12 oder 13 zur Herstellung von Pflege- und Reinigungsformulierungen, insbesondere für Haut und Hautanhangsgebilden und für Haushalt und Industrie, insbesondere textilweichmachende Formulierungen und textilpflegende Wasch- oder Reinigungsmitteln.

15. Pflege- und Reinigungsformulierungen, insbesondere für Haut und Hautanhangsgebilden und für Haushalt und Industrie, insbesondere kosmetische Formulierung, textilweichmachende Formulierungen und textilpflegende Wasch- oder Reinigungsmitteln, enthaltend mindestens eine Mikroemulsion gemäß mindestens einem der Ansprüche 1 bis 11 oder mindestens eine Mikroemulsion erhältlich nach einem Verfahren gemäß Anspruch 12 oder 13, wobei diese bevorzugt ausgewählt sind aus der Gruppe der Haarbehandlungsmittel und Haarnachbehandlungsmittel zum Ausspülen oder zum Verbleib im Haar, Pflege- und Reinigungsformulierungen für Wasserfahr-, Fahr- und Flugzeuge sowie Weichspülmittel.

16. Verwendung mindestens einer Mikroemulsion gemäß mindestens einem der Ansprüche 1 bis 11 oder mindestens eine Mikroemulsion erhältlich nach einem Verfahren gemäß Anspruch 12 oder 13 zur Additivierung von Lacken und Farben, insbesondere zur Beschichtung von Textil, Metall, Leder, Kunststoff, Papier, Pappe und Holz.

17. Lacke und Farben, insbesondere zur Beschichtung von Textil, Metall, Leder, Kunststoff, Papier, Pappe und Holz, bevorzugt als Bindemittel eine wässrige Polyurethandispersion enthaltend,
enthaltend mindestens eine Mikroemulsion gemäß mindestens einem der Ansprüche 1 bis 11 oder mindestens eine Mikroemulsion erhältlich nach einem Verfahren gemäß Anspruch 12 oder 13,.

18. Formulierung gemäß Anspruch 15 oder Lacke und Farben nach Anspruch 17 enthaltend die Mikroemulsion in einer Menge von 0,1 Gew.-% bis 99 Gew.-%, wobei sich die Gew.-% auf die Gesamtformulierung beziehen.

## Claims

1. Microemulsion comprising, as component substantially forming the oil phase,
A) polysiloxane containing at least one quaternary ammonium group and of the general formula (I)
Mₐ M*'*ₐ₁ M*"*ₐ₂ M*'"*ₐ₃ D_{b} D*'*_{b1} D*"*_{b2} D*'"* _{b3} T_{c} T*'*_{c1} Q_{d} formula (I),
where
M = (R¹₃ Si O_{1/2})
M*'* = (R²R¹₂ Si O_{1/2})
M*"* = (R³R¹₂ Si O_{1/2})
M*'"* = (R⁴R¹₂ Si O_{1/2})
D = (R¹₂ Si O_{2/2})
D*'* = (R²R¹ Si O_{2/2})
D*"* = (R³R¹ Si O_{2/2})
D*'"* = (R⁴R¹ Si O_{2/2})
T = (R⁵ Si O_{3/2})
T*'* = (R² Si O_{3/2})
Q = (Si O_{4/2})
a = 0 to 32; preferably 0 to 22, in particular 0 to 12;
a1 = 0 to 10, preferably 0 to 5, in particular 0;
a2 = 0 to 32; preferably 0 to 22, in particular 1 to 12;
a3 = 0 to 10; preferably 0 to 5, in particular 0;
with the proviso that
a + a1 + a2 + a3 ≥ 3, preferably > 3;
b = 20 to 400;
b1 = 0 to 10, preferably 0 to 5, in particular 0;
b2 = 0 to 80, preferably 0 to 50, in particular 0 to 10;
b3 = 0 to 20, preferably 0 to 10, in particular 0;
c = 0 to 30, preferably 1 to 20, in particular 2 to 15;
c1 = 0 to 10, preferably 0 to 5, in particular 0;
d = 0 to 15, preferably 1 to 12, in particular 2 to 10;
with the proviso that
a2 + b2 ≥ 1, preferably ≥ 3, in particular > 3 and
c + c1 + d ≥ 1, preferably > 1, in particular ≥ 3;
R¹ methyl;
R² = independently of one another identical or different alkoxy or acyloxy radicals, such as for example methoxy, ethoxy, n-propoxy or isopropoxy radicals, acetoxy, in particular ethoxy or isopropoxy radicals;
R³ = independently of one another identical or different organic radicals which carry quaternary ammonium functions;
R⁴ = independently of one another identical or different organic epoxy radicals;
R⁵ = independently of one another identical or different radicals R¹, R³ or R⁴, preferably R¹, in particular methyl, phenyl, dodecyl or hexadecyl.

2. Microemulsion according to Claim 1, **characterized in that** it comprises a polysiloxane where R⁴ is identical or different radicals selected from the group

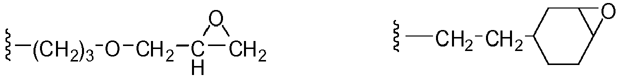

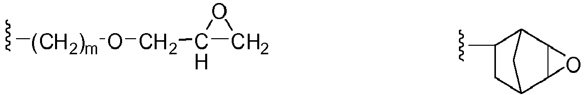

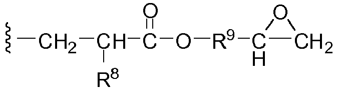

3. Microemulsion according to Claim 1, **characterized in that** it comprises a polysiloxane where
R³ is groups with the structure -R⁶-R⁷, in which
R⁶ are preferably identical or different divalent radicals selected from the group 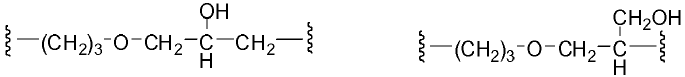 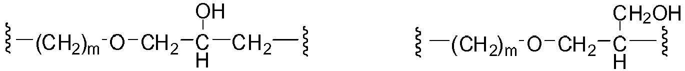 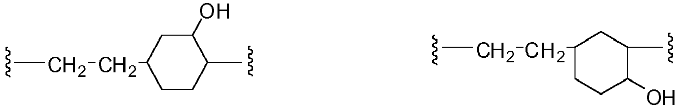 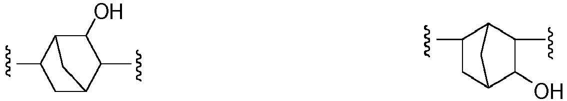 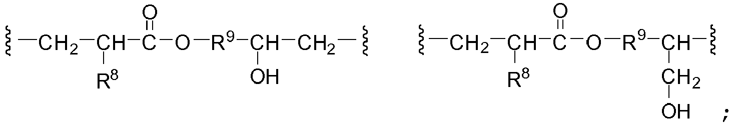
R⁷ is selected from the group consisting of 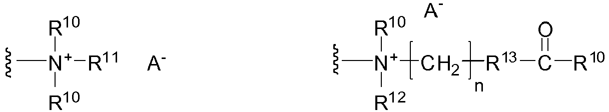 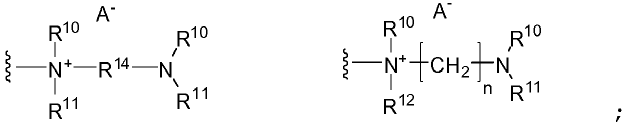
R⁸ are identical or different radicals from the group hydrogen or alkyl having 1 to 6 carbon atoms, preferably methyl;
R⁹ are identical or different divalent hydrocarbon radicals which optionally contain ether functions, preferably methylene;
R¹⁰, R¹¹, R¹² are in each case independently of one another hydrogen or alkyl radicals having 1 to 30 carbon atoms or radicals of the formula 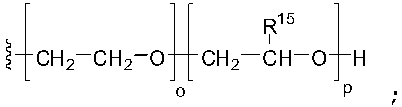
R¹³ are identical or different radicals from the group
-O-; -NR¹⁶-;
R¹⁴ are identical or different optionally branched divalent hydrocarbon radicals, preferably ethylene or propylene;
R¹⁵ are identical or different alkyl, aryl or alkaryl radicals having 1 to 30 carbon atoms which optionally contain ether functions, preferably methyl, ethyl or phenyl, in particular methyl;
R¹⁶ are identical or different radicals from the group hydrogen or alkyl having 1 to 6 carbon atoms;
m = 2 to 18;
n = 2 to 18, preferably 3;
o = 0 to 30, preferably 0 to 10, in particular 1 to 3;
p = 0 to 30, preferably 0 to 10;
A⁻ are identical or different counterions to the positive charges on the quaternized nitrogen groups, selected from inorganic or organic anions of the acids HA, and derivatives thereof.

4. Microemulsion according to at least one of the preceding claims, **characterized in that** it comprises a polysiloxane where
c = 1 and
c + c1 + d = 1.

5. Microemulsion according to at least one of the preceding claims, **characterized in that** the polysiloxane has an average molecular weight of greater than 4000 g/mol, preferably of greater than 7000 g/mol, in particular of greater than 10 000 g/mol.

6. Microemulsion according to at least one of the preceding claims, **characterized in that** it additionally comprises at least one of the components
B) a nonionic surfactant
C) a cosurfactant selected from the group comprising, preferably consisting of, anionic, cationic and amphoteric surfactants and
D) water.

7. Microemulsion according to at least one of the preceding claims, **characterized in that** it is essentially free from alkoxylated compounds.

8. Microemulsion according to Claim 7, **characterized in that** component B) is selected from the group consisting of
glycerol mono- and diesters and sorbitan mono- and diesters of saturated and unsaturated fatty acids, alkyl mono- and oligoglycosides, partial esters based on linear, branched, unsaturated or saturated fatty acids,
ricinoleic acid, and 12-hydroxystearic acid and glycerol, polyglycerol, pentaerythritol, dipentaerythritol, sugar alcohols (e.g. sorbitol), alkylglucosides (e.g. methylglucoside, butylglucoside, laurylglucoside), and polyglucosides (e.g. cellulose),
mono-, di- and trialkyl phosphates and salts thereof,
citric acid esters such as e.g. glyceryl stearate citrate, glyceryl oleate citrate and dilauryl citrate, and glyceryl caprylate, polyglycerylcaprylate, polyglycerylcaprate and mixtures of these surfactants.

9. Microemulsion according to at least one of the preceding claims, **characterized in that** additionally a component E), solvent, is present selected from the group comprising hydrotropes, for example from the group of aliphatic alcohols, such as ethanol, propanol or 1,3-propanediol, cyclic carbonates such as ethylene carbonate, propylene carbonate, glycerol carbonate, esters of mono- or polycarboxylic acids such as ethyl acetate, ethyl lactate, glycerol, isopropyl alcohol, dipropylene glycol, glycol ether and polyols.

10. Microemulsion according to at least one of the preceding claims, **characterized in that** additionally a component F), preservative, and/or a component G), an oil or oil mixture, is present.

11. Microemulsion according to at least one of the preceding claims, **characterized in that**
component A) is present in an amount of from 10% by weight to 60% by weight;
component B) is present in an amount of from 3% by weight to 30% by weight;
component C) is present in an amount of from 0% by weight to 30% by weight;
component D) is present in an amount of from 10% by weight to 75% by weight;
component E) is present in an amount of from 0% by weight to 35% by weight;
component F) is present in an amount of from 0% by weight to 1% by weight and
component G) is present in an amount of from 0% by weight to 50% by weight of the total oil phase consisting of A) and B),
where the % by weight, apart from in the case of component G), refer to the total microemulsion.

12. Process for the preparation of a microemulsion according to at least one of the preceding claims, comprising the process steps
I) provision of at least one polysiloxane as described above,
II) optional dissolution of the polysiloxane with at least one solvent E) and/or a nonionic surfactant of component B) and/or oil G)
III) addition of the remaining components forming the microemulsion, the components water and preservative being added last.

13. Process according to Claim 12, **characterized in that** mixing is carried out during process steps I) to III) with a simple stirrer.

14. Use of at least one microemulsion according to at least one of Claims 1 to 11 or at least one microemulsion obtainable according to a process according to Claim 12 or 13 for producing care and cleaning formulations, in particular for skin and skin appendages and for the home and industry, in particular textile-softening formulations and textile-care detergents or cleaners.

15. Care and cleaning formulations, in particular for skin and skin appendages and for the home and industry, in particular cosmetic formulations, textile-softening formulations and textile-care detergents or cleaners, comprising at least one microemulsion according to at least one of Claims 1 to 11 or at least one microemulsion obtainable by a process according to Claim 12 or 13, where these are preferably selected from the group of hair treatment compositions and hair aftertreatment compositions for rinsing out or for leaving in the hair, care and cleaning formulations for waterborne vessels, vehicles and aircraft, and fabric softeners.

16. Use of at least one microemulsion according to at least one of Claims 1 to 11 or at least one microemulsion obtainable by a process according to Claim 12 or 13 for the additization of coatings and paints, in particular for the coating of textile, metal, leather, plastic, paper, cardboard and wood.

17. Coatings and paints, in particular for the coating of textile, metal, leather, plastic, paper, cardboard and wood, preferably comprising, as binder, an aqueous polyurethane dispersion comprising at least one microemulsion according to at least one of Claims 1 to 11 or at least one microemulsion obtainable according to a process according to Claim 12 or 13.

18. Formulation according to Claim 15 or coatings and paints according to Claim 17 comprising the microemulsion in an amount of from 0.1% by weight to 99% by weight, where the % by weight refer to the total formulation.

## Revendications

1. Microémulsion contenant en tant que composant formant principalement la phase huileuse :
A) au moins un polysiloxane contenant un groupe ammonium quaternaire de formule générale (I)
MₐM*'* ₐ₁M*"* ₐ₂M*'"* ₐ₃D_{b}D*'* _{b1}D*"* _{b2}D*'"* _{b3}T_{c}T*'*_{c1}Q_{d} Formule (I)
dans laquelle
M = (R¹₃SiO_{1/2})
M*'* = (R²R¹₂SiO_{1/2})
M*"* = (R³R¹₂SiO_{1/2})
M*'"* = (R⁴R¹₂SiO_{1/2})
D = (R¹₂SiO_{2/2})
D*'* = (R²R¹SiO_{2/2})
D*"* = (R³R¹SiO_{2/2})
D*'"* = (R⁴R¹SiO_{2/2})
T = (R⁵SiO_{3/2})
T*'* = (R²SiO_{3/2})
Q = (SiO_{4/2})
a = 0 à 32, de préférence 0 à 22, notamment 0 à 12 ;
a1 = 0 à 10, de préférence 0 à 5, notamment 0 ;
a2 = 0 à 32, de préférence 0 à 22, notamment 1 à 12 ;
a3 = 0 à 10, de préférence 0 à 5, notamment 0 ;
à condition que
a + a1 + a2 + a3 ≥ 3, de préférence > 3 ;
b = 20 à 400 ;
b1 = 0 à 10, de préférence 0 à 5, notamment 0 ;
b2 = 0 à 80, de préférence 0 à 50, notamment 0 à 10 ;
b3 = 0 à 20, de préférence 0 à 10, notamment 0 ;
c = 0 à 30, de préférence 1 à 20, notamment 2 à 15 ;
c1 = 0 à 10, de préférence 0 à 5, notamment 0 ;
d = 0 à 15, de préférence 1 à 12, notamment 2 à 10 ;
à condition que
a2 + b2 ≥ 1, de préférence ≥ 3, notamment > 3, et
c + c1 + d ≥ 1, de préférence > 1, notamment ≥ 3 ;
R¹ = méthyle ;
les R² = indépendamment les uns des autres, des radicaux alcoxy ou acyloxy identiques ou différents, tels que par exemple des radicaux méthoxy, éthoxy, n-propoxy ou isopropoxy, des radicaux acétoxy, notamment éthoxy ou isopropoxy ;
les R³ = indépendamment les uns des autres, des radicaux organiques identiques ou différents, qui portent des fonctions ammonium quaternaires ;
les R⁴ = indépendamment les uns des autres, des radicaux époxy organiques identiques ou différents,
les R⁵ = indépendamment les uns des autres, des radicaux R¹, R³ ou R⁴ identiques ou différents, de préférence R¹, notamment méthyle phényle, dodécyle ou hexadécyle.

2. Microémulsion selon la revendication 1, **caractérisée en ce qu'**elle contient un polysiloxane avec les R⁴ = des radicaux identiques ou différents choisis dans le groupe constitué par :

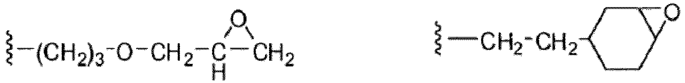

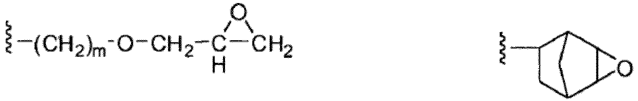

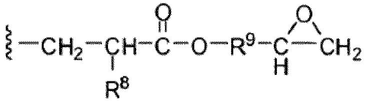

3. Microémulsion selon la revendication 1, **caractérisée en ce qu'**elle contient un polysiloxane avec les R³ = des groupes de structure -R⁶-R⁷, dans laquelle les R⁶ = de préférence des radicaux bivalents identiques ou différents, choisis dans le groupe constitué par : 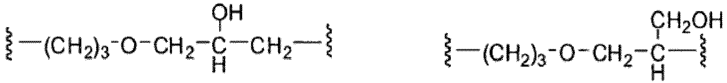 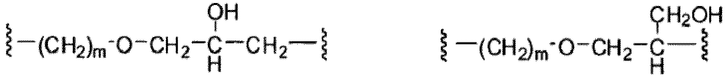 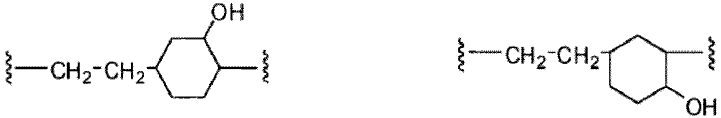 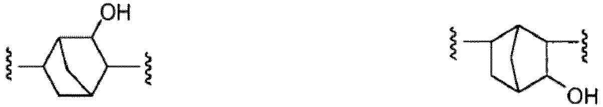 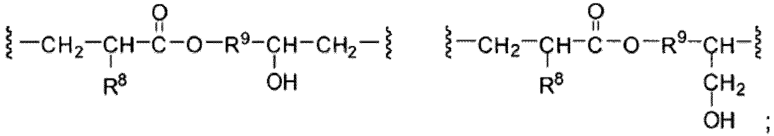
R⁷ est choisi dans le groupe constitué par : 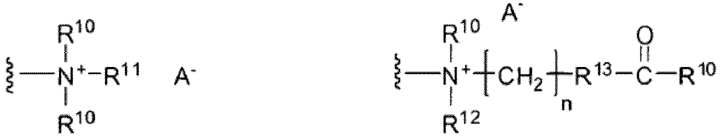 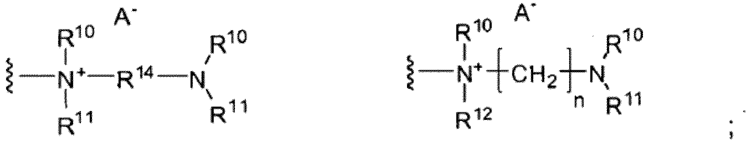
les R⁸ = des radicaux identiques ou différents du groupe constitué par l'hydrogène ou un alkyle de 1 à 6 atomes C, de préférence méthyle ;
les R⁹ = des radicaux hydrocarbonés bivalents identiques ou différents, qui contiennent éventuellement des fonctions éther, de préférence méthylène ;
R¹⁰, R¹¹, R¹² = chacun indépendamment les uns des autres l'hydrogène ou des radicaux alkyle de 1 à 30 atomes C ou des radicaux de formule 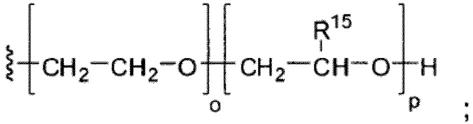
les R¹³ = des radicaux identiques ou différents du groupe constitué par -O- ; -NR¹⁶- ;
les R¹⁴ = des radicaux hydrocarbonés bivalents éventuellement ramifiés identiques ou différents, de préférence éthylène ou propylène ;
les R¹⁵ = des radicaux alkyle, aryle ou alkaryle de 1 à 30 atomes C identiques ou différents, qui contiennent éventuellement des fonctions éther, de préférence méthyle, éthyle ou phényle, notamment méthyle ;
les R¹⁶ = des radicaux identiques ou différents du groupe constitué par l'hydrogène ou un alkyle de 1 à 6 atomes C ;
m = 2 à 18 ;
n = 2 à 18, de préférence 3 ;
o = 0 à 30, de préférence 0 à 10, notamment 1 à 3 ;
p = 0 à 30, de préférence 0 à 10 ;
les A⁻ = des contre-ions identiques ou différents pour les charges positives sur les groupes azote quaternisés, choisis parmi les anions inorganiques ou organiques des acides HA, ainsi que leurs dérivés.

4. Microémulsion selon au moins l'une quelconque des revendications précédentes, **caractérisée en ce qu'**elle contient un polysiloxane avec
c = 1 et
c + c1 + d = 1.

5. Microémulsion selon au moins l'une quelconque des revendications précédentes, **caractérisée en ce que** le polysiloxane présente un poids moléculaire moyen supérieur à 4 000 g/mol, de préférence supérieur à 7 000 g/mol, notamment supérieur à 10 000 g/mol.

6. Microémulsion selon au moins l'une quelconque des revendications précédentes, **caractérisée en ce qu'**elle contient en outre au moins un des composants :
B) un tensioactif non ionique,
C) un co-tensioactif choisi dans le groupe contenant, de préférence constitué par, les tensioactifs anioniques, cationiques et amphotères, et
D) de l'eau.

7. Microémulsion selon au moins l'une quelconque des revendications précédentes, **caractérisée en ce qu'**elle est essentiellement exempte de composés alcoxylés.

8. Microémulsion selon la revendication 7, **caractérisée en ce que** le composant B) est choisi dans le groupe constitué par
les mono- et diesters de glycérine et les mono- et diesters de sorbitane d'acides gras saturés et insaturés,
les mono- et oligoglycosides d'alkyle, les esters partiels à base d'acides gras linéaires, ramifiés, insaturés ou saturés,
l'acide ricinoléique, ainsi que l'acide 12-hydroxystéarique et la glycérine, la polyglycérine, la pentaérythrite, la dipentaérythrite, les alcools de sucre (p. ex. sorbitol), les alkylglucosides (p. ex. méthylglucoside, butylglucoside, laurylglucoside), ainsi que les polyglucosides (p. ex. cellulose),
les phosphates de mono-, di- et trialkyle et leurs sels, les esters de l'acide citrique, tels que p. ex. le stéarate citrate de glycéryle, l'oléate citrate de glycéryle et le citrate de dilauryle, ainsi que le caprylate de glycéryle, le polycaprylate de glycéryle, le polycaprate de glycéryle,
et les mélanges de ces tensioactifs.

9. Microémulsion selon au moins l'une quelconque des revendications précédentes, **caractérisée en ce qu'**elle contient en outre un composant E), un solvant, choisi dans le groupe contenant :
les hydrotropes, par exemple du groupe constitué par les alcools aliphatiques, tels que l'éthanol, le propanol ou le 1,3-propanediol, les carbonates cycliques, tels que le carbonate d'éthylène, le carbonate de propylène, le carbonate de glycérine, les esters d'acides mono- ou polycarboxyliques tels que l'acétate d'éthyle, le lactate d'éthyle, la glycérine, l'alcool isopropylique, le dipropylène glycol, les éthers de glycol et les polyols.

10. Microémulsion selon au moins l'une quelconque des revendications précédentes, **caractérisée en ce qu'**elle contient en outre un composant F), un conservateur, et/ou un composant G), une huile ou un mélange d'huiles.

11. Microémulsion selon au moins l'une quelconque des revendications précédentes, **caractérisée en ce que** le composant A) est contenu en une quantité de 10 % en poids à 60 % en poids,
le composant B) est contenu en une quantité de 3 % en poids à 30 % en poids,
le composant C) est contenu en une quantité de 0 % en poids à 30 % en poids,
le composant D) est contenu en une quantité de 10 % en poids à 75 % en poids,
le composant E) est contenu en une quantité de 0 % en poids à 35 % en poids,
le composant F) est contenu en une quantité de 0 % en poids à 1 % en poids, et
le composant G) est contenu en une quantité de 0 % en poids à 50 % en poids de la phase huileuse totale constituée par A) et G),
les % en poids se rapportant à la microémulsion totale, sauf dans le cas du composant G).

12. Procédé de fabrication d'une microémulsion selon au moins l'une quelconque des revendications précédentes, comprenant les étapes de procédé suivantes :
I) la préparation d'au moins un polysiloxane tel que décrit précédemment,
II) éventuellement la dissolution du polysiloxane avec au moins un solvant E) et/ou un tensioactif non ionique selon le composant B) et/ou une huile G),
III) l'ajout du reste des composants formant la microémulsion,
les composant eau et conservateur étant ajoutés en dernier.

13. Procédé selon la revendication 12, **caractérisé en ce qu'**un mélange avec un agitateur simple a lieu pendant les étapes de procédé I) à III).

14. Utilisation d'au moins une microémulsion selon au moins l'une quelconque des revendications 1 à 11 ou d'au moins une microémulsion pouvant être obtenue par un procédé selon la revendication 12 ou 13 pour la fabrication de formulations de soin et de nettoyage, notamment pour la peau et les annexes de la peau, et pour la maison et l'industrie, notamment de formulations assouplissantes pour textiles et de détergents pour le soin des textiles.

15. Formulations de soin et de nettoyage, notamment pour la peau et les annexes de la peau, et pour la maison et l'industrie, notamment formulations cosmétiques, formulations assouplissantes pour textiles et détergents pour le soin des textiles, contenant au moins une microémulsion selon au moins l'une quelconque des revendications 1 à 11 ou d'au moins une microémulsion pouvant être obtenue par un procédé selon la revendication 12 ou 13, celles-ci étant de préférence choisies dans le groupe constitué par les agents de traitement des cheveux et les agents de traitement ultérieur des cheveux destinés à être rincés ou à rester dans les cheveux, les formulations de soin et de nettoyage pour véhicules marins, véhicules et véhicules aériens, ainsi que les assouplissants.

16. Utilisation d'au moins une microémulsion selon au moins l'une quelconque des revendications 1 à 11 ou d'au moins une microémulsion pouvant être obtenue par un procédé selon la revendication 12 ou 13 en tant qu'additif dans des vernis et des peintures, notamment pour le revêtement de textiles, de métaux, de cuir, de plastiques, de papier, de carton et de bois.

17. Vernis et peintures, notamment pour le revêtement de textiles, de métaux, de cuir, de plastiques, de papier, de carton et de bois, de préférence contenant en tant que liant une dispersion aqueuse de polyuréthane, contenant au moins une microémulsion selon au moins l'une quelconque des revendications 1 à 11 ou au moins une microémulsion pouvant être obtenue par un procédé selon la revendication 12 ou 13.

18. Formulation selon la revendication 15 ou vernis et peintures selon la revendication 17, contenant la microémulsion en une quantité de 0,1 % en poids à 99 % en poids, les % en poids se rapportant à la formulation totale.
